# EUROPEAN PATENT APPLICATION

(11) **EP 4 730 663 A1**
(43) Date of publication of application: **22.04.2026**
(21) Application number: 25207934.8
(22) Date of filing: 10.10.2025
(51) Int. Cl.: H04B 1/3827, A61B 5/00

(54) **METHODS AND SYSTEMS FOR MULTI-LAYER CONTROL OF TRANSMITTER ACTIVITY**

(30) Priority: 16.10.2024 US 202418917657
(71) Applicant: GE Precision Healthcare LLC, Waukesha, WI 53188 (US)
(72) Inventor: HAKALA, Timo, 00510 Uusimaa (FI); LAINE, Tuomas, 00510 Uusimaa (FI)
(74) Representative: Kilburn & Strode LLP

(57) **Abstract**

Systems and methods are provided for multi-layer control of transmitter activity. In one example, a method for a transmitter includes adjusting output of the transmitter based on each of one or more measured duty cycles relative to one or more respective thresholds, wherein the adjusting includes adjusting output of the transmitter at a physical or data link layer, adjusting output of the transmitter by adjusting a data rate of data released to the transmitter, and/or adjusting output of the transmitter by adjusting a data flow to the transmitter.

## Description

### TECHNICAL FIELD

Embodiments of the subject matter disclosed herein relate wireless transmitters and, more particularly, to control of wireless transmitter duty cycle.

### BACKGROUND

Wireless communication comprises one or more radio transmitters sending radio signals and one or more radio receivers receiving radio signals. A radio transmitter may include an antenna to transmit radio signals, as well as one or more processors to prepare a signal for transmission. Radio transmitters may be included in devices such as cell phones, aircraft radios, garage door openers, Bluetooth devices, and devices connected to wireless local area networks (WLAN) such as Wi-Fi networks. Transmitters may not operate continuously, and the portion of time the transmitter spends operating may be referred to as the transmission duty cycle.

In some examples, radio transmitters may be used within a healthcare setting, such as a hospital. In a healthcare setting, clinicians may monitor physiological parameters of a patient obtained with one or multiple sensors. Some patient monitoring systems may include a patient monitor capable of wirelessly connecting to one or more sensors in order to collect, process, and/or display physiological information describing the patient's condition. The patient monitor may include one or more radio transmitters that allow the patient monitor to be connected to a wireless network, such as Wi-Fi, within the hospital environment.

### SUMMARY

In one example, a method for a transmitter includes measuring a duty cycle of the transmitter over each of one or more time periods, and adjusting output of the transmitter based on each measured duty cycle relative to one or more respective thresholds, wherein the adjusting includes adjusting output of the transmitter at a physical or data link layer, adjusting output of the transmitter by adjusting a data rate of data released to the transmitter, and/or adjusting output of the transmitter by adjusting a data flow to the transmitter.

It should be understood that the summary above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

Various aspects of this disclosure may be better understood upon reading the following detailed description and upon reference to the drawings in which:
FIG. 1 schematically illustrates a patient monitor within a patient monitoring environment.
FIG. 2 shows the patient monitor of FIG. 1 in communication with a sensor system.
FIG. 3 is a schematic block diagram of a patient monitoring system including the patient monitor of FIG. 1 connected to one or more networks.
FIG. 4 is schematic diagram of the layers of the open systems interconnection (OSI) model including an application layer, a network layer, a link layer, a physical layer, and other layers.
FIG. 5 is a schematic diagram of the flow of data through the application layer, the network layer, the data link layer, and the physical layer of the OSI model within the patient monitor of FIG. 1.
FIG. 6 is a plot of the transmission activity of a transmitter such as the patient monitor of FIG. 1 over time.
FIG. 7 is a high-level flowchart depicting a method for adjusting the data flow through one or more layers of the OSI model of a transmitting device, such as the patient monitor of FIG. 1, based on one or more measured duty cycles of the transmitting device.
FIG. 8 is a flowchart depicting a method for adjusting the data flow through the physical and/or or link layer of the transmitting device based on two measured duty cycles.
FIG. 9 is a flowchart depicting a method for adjusting a data rate through the network layer of the transmitting device based on a measured duty cycle.
FIG. 10 is a flowchart depicting a method for adjusting data flow through the application layer of the transmitting device based on a measured duty cycle.

### DETAILED DESCRIPTION

The following relates to systems and methods for monitoring and managing a duty cycle of a radio transmitter device. Radio transmitter devices may include devices such as cell phones, tablets, and other wireless devices. For example, a radio transmitter device may be included within a wireless patient monitoring system. Patient monitoring using the wireless patient monitoring system may include receiving physiological patient data from one or more sensors placed on a patient and displaying the physiological patient data on a patient monitor and/or uploading the physiological patient data to hospital information systems via a wireless network. Once the physiological patient data has been uploaded to the hospital information systems, the physiological patient data may be transmitted to other devices such as a caregiver device for remote viewing.

Thus, the wireless patient monitoring system may include one or more physiological monitoring devices, sensors, etc., capable of monitoring cardiac, respiratory, neurologic, hemodynamic, pulse oximetry, and/or other parameters including but not limited to electrocardiography (ECG), oxygen saturation (SpO2), respiration rate, temperature, blood pressures, entropy, blood glucose, and carbon dioxide. The physiological monitoring device(s) may be connected to the wireless network via the patient monitor, which may be a computing device in the form of a mobile device that includes a wireless transmitter. The patient monitor may be configured to transmit patient monitoring data received from the physiological monitoring device(s), via the wireless network, to the hospital information system. Thus the patient monitor may be an example of a radio transmitter device.

Regulatory limits are frequently imposed upon the transmission duty cycle of the radio transmitter within a radio transmitter device. The transmission duty cycle of the radio transmitter is a measure of the fraction of time during which the transmit signal power is active. Regulatory limits may be placed on the specific absorption rate (SAR) allowed for people in contact with the radio transmitter. The SAR depends on the energy radiated by the radio transmitter, which depends directly upon the transmission duty cycle of the radio transmitter among other factors. However, the transmission duty cycle depends on a plurality of factors including the data being transmitted by the radio transmitter and the environment, and may be difficult to determine reliably. In that case, regulatory compliance may be based on the assumption that the transmission duty cycle is continuous (e.g., 100%), which does not accurately reflect the operation of the radio transmitter and imposes unnecessary constraints on the design of radio transmitter devices. Additional upper boundaries may be placed on the transmission duty cycle due to wireless coexistence issues. For example, internal blocking occurs when an active transmitter prevents co-located receivers from receiving on all channels. External collision occurs when an active transmitter prevents nearby devices from receiving data at the channel the transmitter is using to transmit data. Internal coexistence and external collision depend directly on the transmission duty cycle of the radio transmitter.

In the example that the radio transmitter is included in the patient monitoring system, one or more patient monitoring systems may be active in a healthcare setting simultaneously. The patient monitoring systems may encounter issues due to internal coexistence and external collision, which may impact the transmission and reception of data from the patient monitoring system.

Thus, according to embodiments disclosed herein, the duty cycle of a radio transmitter may be repeatedly calculated over one or more time periods. The duty cycle may be measured by integrating the amount of time the radio transmitter is active during the plurality of time periods and dividing each integrated time by a length of a respective time period. The radio transmitter may be determined to be active by a radio frequency (RF) power detector integrated into the radio transmitter. Based on the calculated duty cycles, the transmission of data may be adjusted at one or more open systems interconnection (OSI) model layers, each layer representing a set of functions, methods, and associated hardware used in communication among interconnected computer systems. The OSI model is a model that describes the ways data can be transmitted within a network. The layers of the OSI model are ordered and include, in order from lowest to highest, a physical layer, a data link layer, a network layer, and an application layer, among other layers. For example, the physical layer is where bits of data are transmitted over a physical medium, such as within wires in a device, the data link layer defines the data formats and protocol procedures of data on the communication links, the network layer determines the physical routing path of the data for communication, and the application layer is the layer at which humans interact with the network and where the data to be communicated is generated.

Limiting the data flow at a higher layer of the OSI model is generally least disruptive for the communication but does not correspond to changes in the transmission duty-cycle accurately and quickly. Therefore, the transmission duty cycle may be limited at the highest possible OSI model layer that is appropriate. Thus, in some examples, it may be preferred to limit the data flow out of the application layer than to set a strict data rate (bps) limit at the network layer. Equally it may be preferred to set a data rate limit at the network layer than disrupt the data link layer or the transmission of data through the physical layer. Therefore, duty cycle measured over shorter time periods may be used to adjust lower layers such as the physical layer and link layer, duty cycle measured over intermediate time periods may be used to adjust intermediate layers, such as adjusting the data rate through the network layer, and duty cycle measured over longer time periods may be used to control the higher layers, such as the application layer.

For example, at the application layer, multiple priority-ranked queues may be arranged to transmit high-priority data, and allow wait times for lower-priority data in response to a prompt to reduce data flow through the application layer. In addition, certain types of data may be blocked from transmission, such as backfill data, software updates, and/or waveforms. At the network layer, the data rate may be controlled to reduce transmission using a data rate limiter that may employ strategies such as traffic shaping. The data rate limiter may impose a maximum data rate (in bps) that is allowed to be transmitted by the network layer. There may be a latency in the control of the transmission duty cycle. At the physical layer, data transmission is only be permitted or blocked, and blocking or permitting data transmission may be accomplished via a switch. Similarly, data transmission at the data link layer may only be permitted or blocked based on protocol data units. There is no or negligible latency in the control of the transmission duty cycle when controls are initiated at the physical layer and/or the data link layer. That is because all RF signals travel to the antenna through the physical layer according to scheduling by the data link layer.

By monitoring the duty cycle of a radio transmitter of a radio transmission device and limiting or permitting the flow of data at a plurality of OSI layers of the radio transmission device in response to the measured duty cycles, higher rates of data transmission may be allowed without exceeding regulatory limits relative to data transmission rates allowed when operating under the assumption that the transmission of data is continuous. The method described above employs multiple layers of duty cycle control to maintain the transmission duty cycle within regulatory limits while providing minimal interruptions to transmission. For example, data flow may be reduced at the application layer based on an increased transmission duty cycle over a longer time span, such as 300 s. Reducing the rate of data flow through the application layer may present little to no interruption to the transmission or reception of high-priority data from a patient monitoring system. Data flow may be further controlled at the network layer based on the duty cycle measured over a medium time span, such as 1 s. Traffic shaping at the network layer may allow the data flow to be reduced through the network layer with little impact on the end users. Data flow may finally be controlled at the data link or physical layers by blocking or allowing data transmission based on the duty cycle measured over a shorter time span, such as 0.1 s and the longer time span used in limiting the application layer. Blocking transmission may cause significant interruptions to the function of the radio transmission device. However, reducing the flow of data at the application and network layers may reduce the demand for blocking transmission at the physical layer. Blocking radio transmission at the physical layer based on duty cycle data collected over the short time span enables the radio transmission device to quickly block transmission if the duty cycle increases to approach regulatory limits. Reducing data flow at the application layer and at the physical layer may minimize interruptions to service and ensure that the transmission duty cycle of the radio transmitter will not exceed regulatory limits.

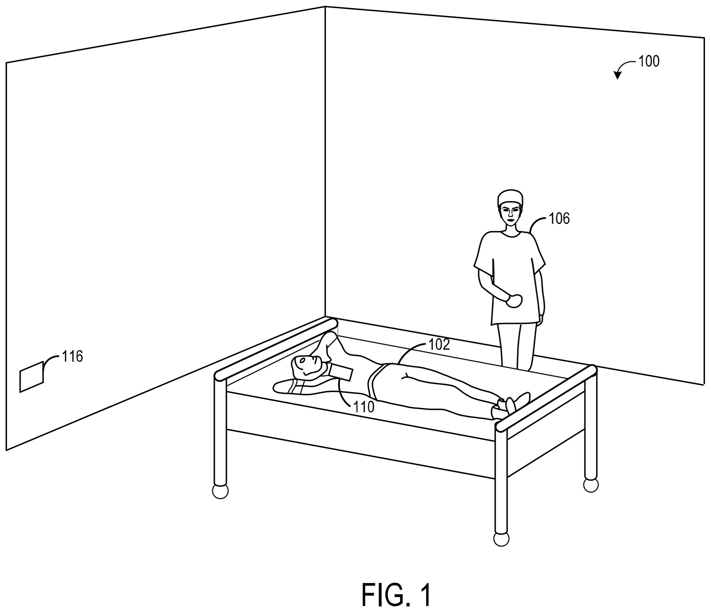

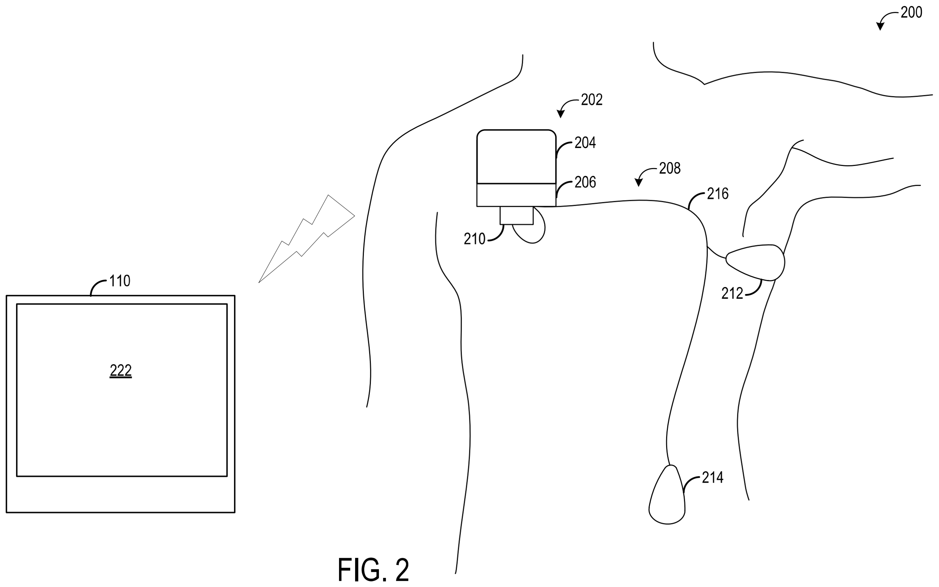

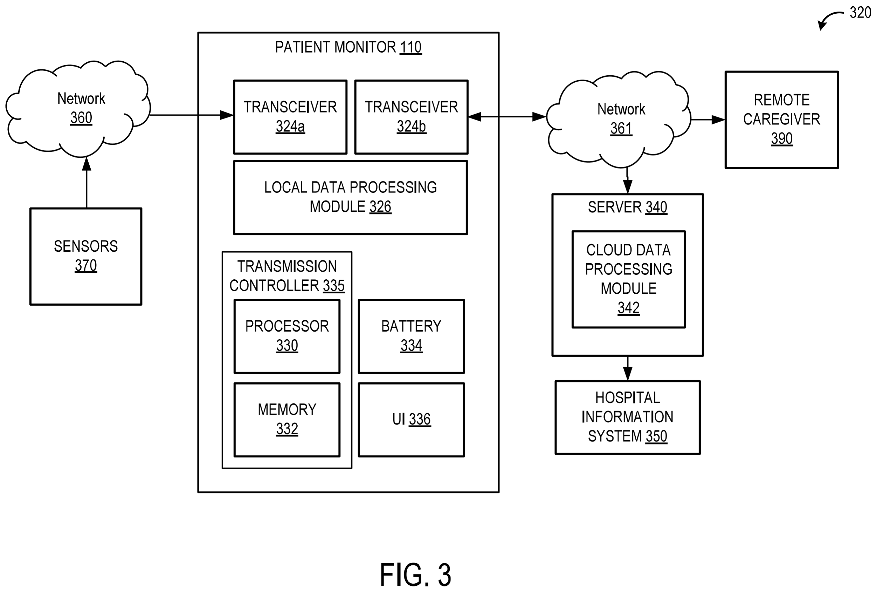

An example patient monitoring system is shown in FIG. 1, and the example patient monitoring system includes a patient monitor coupled to a patient within a patient monitoring environment. FIG. 2 is a diagram of an example patient monitor in wireless communication with a sensor system that collects physiological data from the patient. FIG. 3 shows a schematic diagram of wireless patient monitoring system including a patient monitor, which may monitor a health status of a patient within a patient monitoring environment. Components of the patient monitor used in wireless communication are included within the diagram, as well as wireless connections between the patient monitor and other devices over a network. A caregiver may use a remote monitoring application to communicate with the patient monitor when the clinician is not physically near the patient. Additionally, the patient monitor may receive physiological patient data transmitted from one or more sensors arranged on a body of the patient, and display health status information and/or the physiological patient data on a screen of the patient monitor.

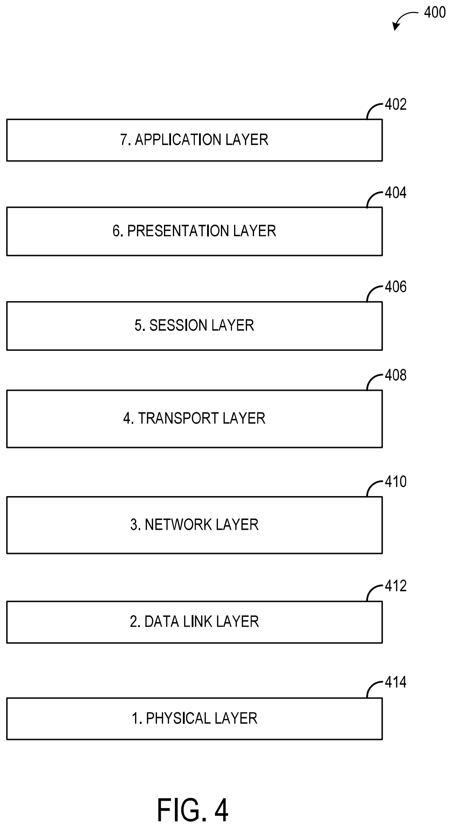

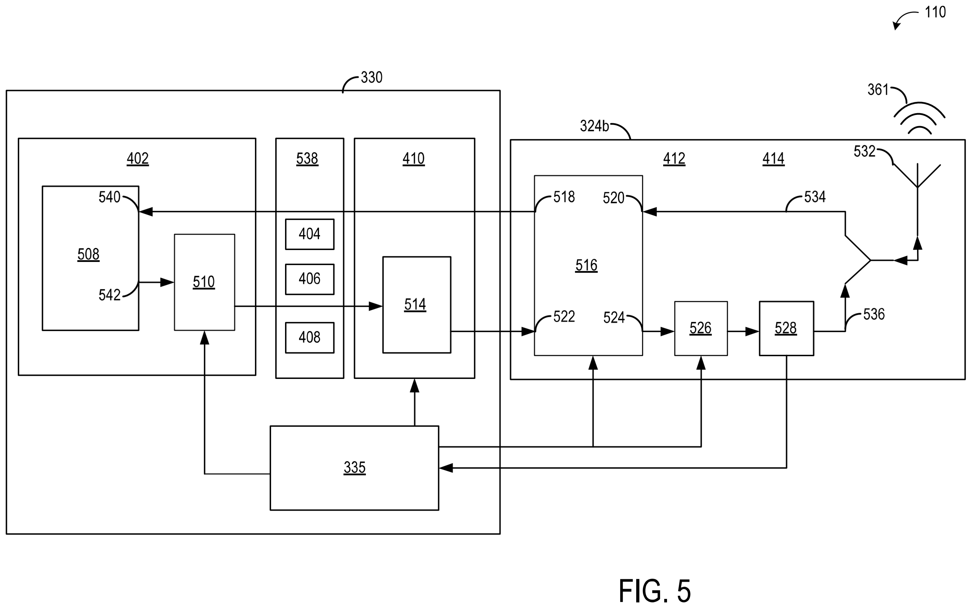

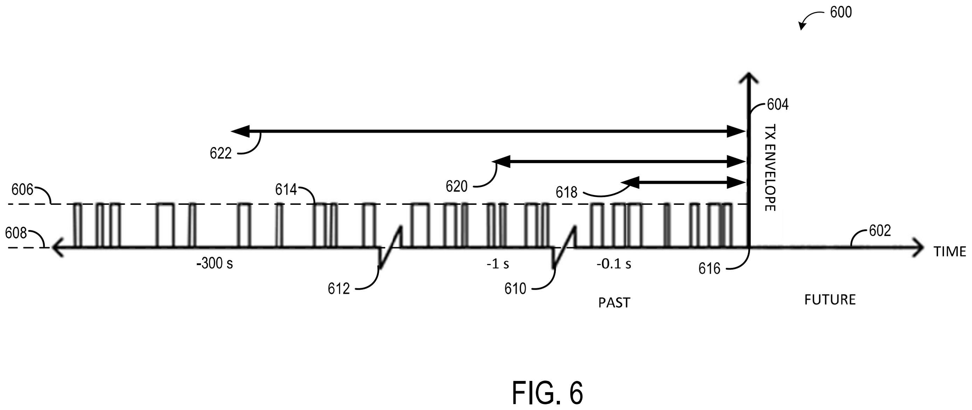

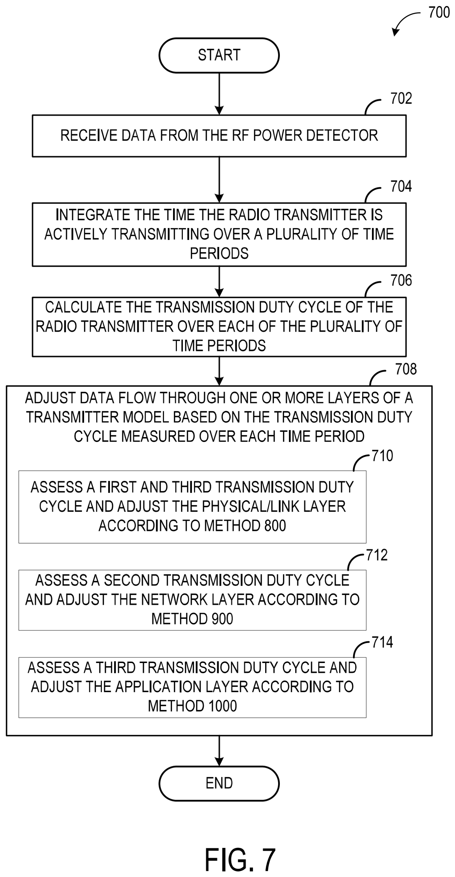

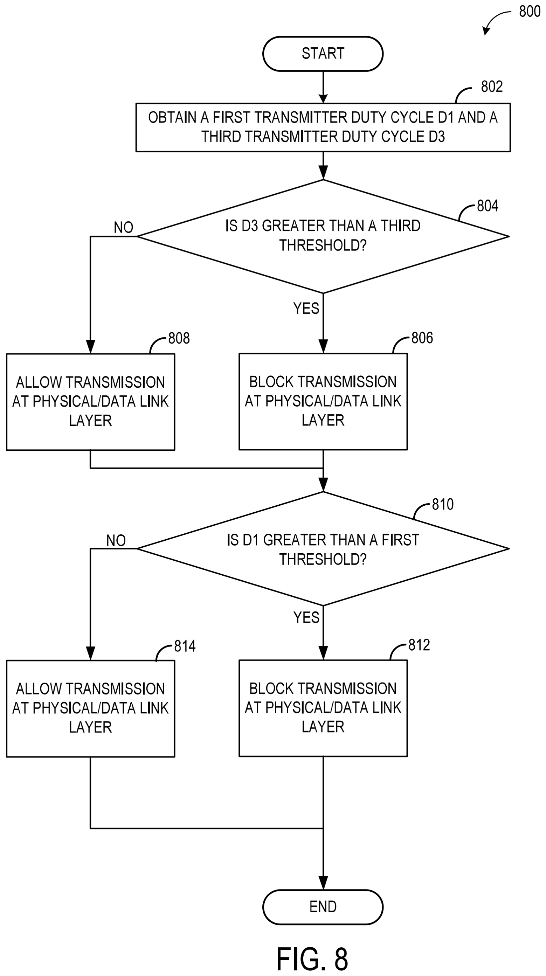

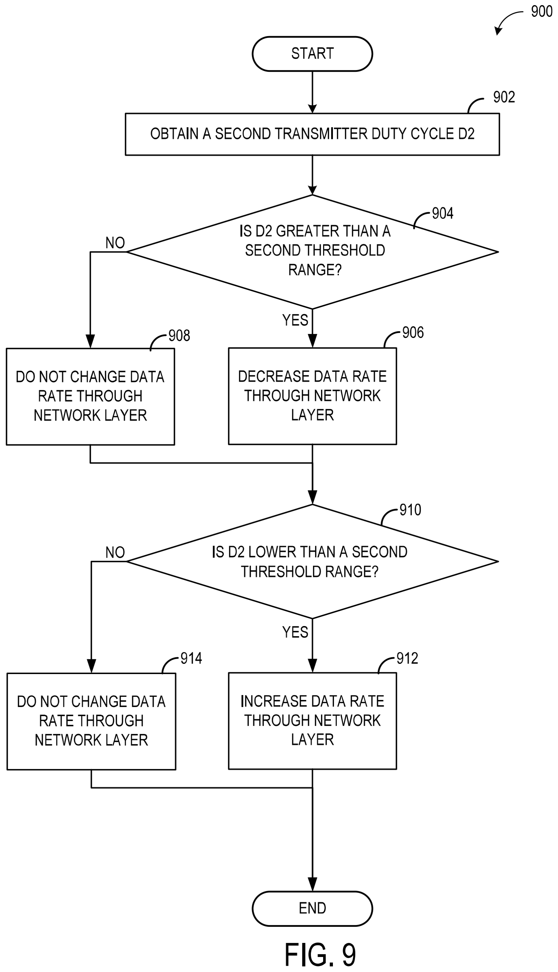

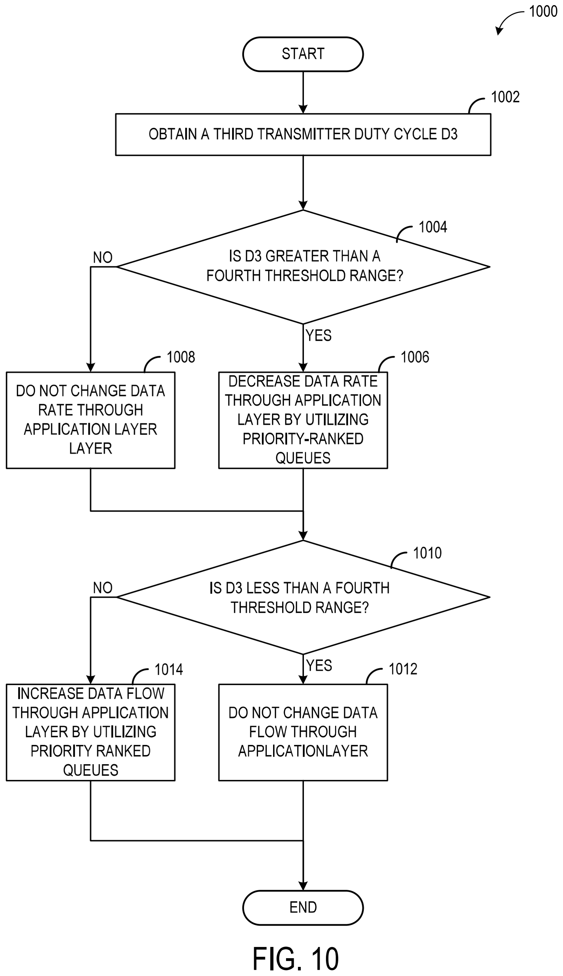

Wireless communication may include a plurality of processes to prepare, transmit, and receive data. These processes may be understood and sorted according to a model of wireless communication. The OSI model is one such model. FIG. 4 is a diagram of the layers of the OSI model including the application layer, the network layer, the data link layer, and the physical layer. FIG. 5 is a schematic diagram of a radio transmitter device, such as the patient monitor, that includes components to control the flow of data through the application layer, the network layer, the physical layer, and the data link layer. The components that control the flow of data through the selected OSI layers may be controlled by a transmission controller of the radio transmitter device. The radio transmitter device may further include a component to measure the transmission envelope of the signal transmitted by the radio transmitter within the radio transmitter device in order to determine the duty cycle, as shown in FIG. 6. FIG. 6 includes three time periods over which the duty cycle of the radio transmitter may be measured. FIG. 7 is a flowchart that includes a method to measure the duty cycle of the radio transmitter of the radio transmitter device over one or more time periods, and use the measured duty cycles to determine how to control the flow of data through the radio transmitter device. FIG. 8 is a flowchart depicting a method for controlling the flow of data through the physical layer and data link layer, FIG. 9 is a flowchart depicting a method for controlling the flow of data through the network layer, and FIG. 10 is a flowchart depicting a method for controlling the flow of data through the application layer.

FIG. 1 shows an example patient monitoring environment 100, which in the depicted example is a patient room in a hospital or other medical facility. The patient monitoring environment 100 may include one or more patient monitoring devices, monitoring one or more physiological parameters. The patient monitoring environment 100 includes a patient 102 being monitored by one or more monitoring devices and also being attended to by a clinician 106. Clinician 106 may be a nurse, physician, medical technologist, or another suitable medical professional. The monitoring devices include a patient monitor 110. The patient monitoring environment 100 depicts the use of one monitoring device but it is understood this is non-limiting as the patient monitoring environment 100 could include one device monitoring one parameter, one device monitoring more than one parameter, multiple devices each monitoring one or more than one parameter, and so on. Due to differing patient conditions and the varying patient monitoring needs, one or more devices may be used to support the monitoring needs of the patient and capable of supporting monitoring in various conditions (e.g., in room, in transport, patient ambulation).

Patient monitor 110 may include one or more telemetry devices (with different sensor capabilities) housed in a common module or housed in two or more separate modules. Patient monitor 110 may be positioned on the patient (e.g., via a pouch, holder, or similar, attached to a belt of the patient) or on a movable module (e.g., a wheeled module), such that patient monitor 110 may leave the patient room if the patient leaves the patient room, and may travel with the patient. Patient monitor 110 may be connected to the patient 102 via one or more leads or other components or in wireless communication with an associated sensor (an example of which is shown in FIG. 2), in order to monitor one or more parameters of the patient (such as ECG, respiration, blood oxygen level, etc.).

The patient monitoring data collected by patient monitor 110 may be sent wirelessly (e.g., a wireless local area network (WLAN) such as Wi-Fi, Bluetooth, Medical Body Area Network (MBAN)) and/or via a hard-wired networked connection to one or more associated devices for processing, analysis, storage, display, etc., such as a central station, patient monitoring database, and/or different patient monitoring system. The methods of wireless communication used by patient monitor 110 and the receiving systems vary widely based on the technology used. In one example communication approach, to facilitate the transfer of the patient monitoring data collected by patient monitor 110, patient monitoring environment 100 and nearby areas (e.g., hallways, closets, open spaces) may include one or more access points 116. Access point 116 may receive and send information (e.g., wirelessly) to patient monitor 110 (e.g., the patient monitoring data, communication status). The access point 116 sends the received information to a processing server, a central station, a telemetry monitoring system, and/or another suitable device. If patient 102 leaves the patient room and moves throughout the medical facility, patient monitoring data collected by patient monitor 110 may be sent to other access points located throughout the medical facility. It is understood that this example using a transceiver and access point 116 for data communications is one of many different technologies suitable for sharing acquired patient monitoring data with the associated data processing, analysis, storage, and information viewing system components and infrastructure.

FIG. 2 shows an example patient monitoring system 200 including a sensor system 202. Sensor system 202 includes a host device 204, a sensor device 206, and a sensor 208. As shown in FIG. 2, the sensor device 206 is mechanically connected to the host device 204 and the sensor 208 is mechanically connected to the sensor device 206.

The sensor system 202 is in the form of a respiration rate monitor, and thus the sensor 208 includes a plurality of electrodes, including electrode 212 and electrode 214, each of which are connected to a sensor base/connector 210 via a cable harness 216 (also referred to as a cable). While not shown in FIG. 2, in some examples, an electrode and/or adhesive patch of the sensor 208 may be positioned behind the host device 204 and/or sensor device 206. The electrodes (and adhesive patch) are shown as being coupled to a chest of a patient.

The host device 204 is configured to communicate wirelessly with the patient monitor 110 and/or a processing system. In some examples, the host device 204 may communicate with the processing system (not shown in FIG. 2; an example processing system in the form of a server or hospital information system is shown in FIG. 3) via the patient monitor 110. The patient monitor 110 may include a display screen 222, via which physiological data collected by sensor 208 may be displayed to a user and/or via which notifications may be displayed to a user.

Referring now to FIG. 3, a patient monitoring system 320 is shown that may be a non-limiting example of the patient monitoring system 200 described with respect to FIG. 2. The patient monitoring system 320 includes the patient monitor 110 of FIG. 1, which may be connected to one or more sensors 370 and a server 340, where patient data may be transmitted from the one or more sensors 370 to patient monitor 110 over a first network 360. The patient monitor 110 may also send data to server 340, and receive data transmitted from server 340 over a second network 361. In some examples, the first network 360 may be the same network as the second network 361. However, in other embodiments the first network 360 may be different from the second network 361. In one example, the first network 360 is a body area network, such as a body area network (BAN) and the second network 361 is a local area network such as WLAN. In various embodiments, the patient monitoring system 320 may be established within a hospital environment or healthcare facility, such as within a patient monitoring environment such as the patient monitoring environment 100 of FIG. 1, and thus the first network 360 may be a medical body area network (MBAN).

The one or more sensors 370 may be specially designed devices for sensing a certain type or types of patient data via placement on a patient's body, and communicating the patient data to patient monitor 110. The one or more sensors 370 may further include a plurality of sensors of different types or the same type. The one or more sensors 370 may include sensors for obtaining physiological patient data from a patient. For example, the one or more sensors may include, but are not limited to, a 3-lead ECG, a pulse oximetry sensor, a blood pressure sensor, a digital stethoscope, a respiratory sensor, a temperature sensor, and the like. The one or more sensors 370 may include a combination of one or more different kinds of sensor. One example of the sensors 370 is the sensor 208 described with respect to FIG. 2.

The physiological patient data is alternatively referred to herein as patient data. The patient data may include, for example, vital signs of the patient, such as a blood pressure or a pulse, and/or any other type of data that may be acquired from a sensor capable of acquiring patient physiological patient data in real-time. Thus, the data sensed by the one or more sensors 370 correlates to the type of sensors in one or more sensors 370 and may include ECG data, PPG data, blood pressure data, SpO2 data, respiratory rate, otoscope data, temperature data, and the like. It will be appreciated that the types of sensors listed above are mentioned for illustrative purposes, and the one or more sensors 370 may additionally include other types of sensors for obtaining physiological patient data of a patient without departing from the scope of this disclosure.

The one or more sensors 370 may communicate the acquired patient data wirelessly to patient monitor 110 over the first network 360, which may include in a non-limiting manner, a BAN, a wide area network (WAN); a local area network (LAN); the Internet; a wired or wireless (e.g. optical, Bluetooth, Bluetooth Low Energy (BLE), radio frequency (RF) network; a cloud-based computer infrastructure of computers, routers, servers, gateways, etc., or any combination thereof associated therewith that allows one or more computing devices within the patient monitoring system 320 to connect with each other. The first network 360 and the second network 361 may be or include a public network, or a private network associated with a portion of a care facility, for example a surgery module or department of a hospital, or may be more broadly located across medical devices of an entire hospital or hospital system. In some embodiments, patient monitor 110 and the one or more sensors 370 may be communicatively coupled via a wireless personal area network (PAN) technology such as MBAN. In other embodiments, any PAN technology may be used, such as induction wireless, infrared wireless, ultra wideband (UWB), Bluetooth^{®}, or any other similar technology for wireless communication between co-located devices. For example, the one or more sensors 370 may communicate with patient monitor 110 via the first network 360 (e.g., via MBAN), and patient monitor 110 may communicate with other elements of patient monitoring system 320 via the second network 361 (e.g., via WLAN).

The patient monitor 110 may include one or more transceivers, such as a first transceiver 324a and a second transceiver 324b, a local data processing module 326, a processor 330, a memory 332, a battery 334, and a user interface (UI) 336. The patient monitor 110 may be adapted to receive data from the first network 360 and the second network 361 via the transceiver(s). In the example that the first network 360 is not the same as the second network 361, the first transceiver 324a is configured to receive and transmit data to and from the first network 360 and the second transceiver 324b is configured to send and receive data to and from the second network 361. The transceiver(s) may therefore refer to the hardware and functions associated with one or more transceivers. In some embodiments, one or more of the transceivers may be or may include a WLAN wireless card. In some embodiments, the WLAN card may be an original equipment manufacturer (OEM) card, and may include a storage medium having computer executable code and a processor to execute that code, thus effectuating the operation of the WLAN card. The second transceiver 324b may connect to the second network 361 via an access point of the network (e.g., access point 116 of FIG. 1) arranged at a location of the hospital environment, for example, in proximity to patients being monitored at a care module. The second network 361 may receive wirelessly transmitted information from the access point, and relay the information to one or more connected devices and/or a hospital information system suitable for collecting and managing such information.

Patient monitor 110 may include a processor 330. The processor 330 may control the operation of patient monitor 110 in response to control signals from the UI 336. In some embodiments, the UI 336 may be integrated into patient monitor 110, where a user may interact with, adjust, or select control elements in the UI 336 (e.g., buttons, knobs, touchscreen elements, etc.) to send one or more control signals to the processor 330 from the UI 336. In other embodiments, the UI 336 is not integrated into patient monitor 110, and the user may interact with, adjust, or select control elements in UI 336 via a user input device, such as a mouse, track ball, touchpad, etc., or the operator may interact with UI 336 via a separate touchscreen, where the operator touches a display screen of UI 336 to interact with UI 336, or via another type of input device.

The UI 336 may include a display (e.g., screen or monitor) and/or other subsystems. Patient monitor 110 may be in the form of a laptop computing device, a tablet, a smart phone, or any other device configured to transmit data over a network. For example, a patient receiving remote care from their home may use a tablet or mobile device as patient monitor 110.

The processor 330 may execute instructions stored on a memory 332 to control patient monitor 110. As discussed herein, the memory 332 may include any non-transitory computer readable medium in which programming instructions are stored. For the purposes of this disclosure, the term "tangible computer readable medium" is expressly defined to include any type of computer readable storage. The example methods and systems may be implemented using coded instruction (e.g., computer readable instructions) stored on a non-transitory computer readable medium such as a flash memory, a read-only memory (ROM), a random-access memory (RAM), a cache, or any other storage media in which information is stored for any duration (e.g. for extended period time periods, permanently, brief instances, for temporarily buffering, and/or for caching of the information). Computer memory of computer readable storage mediums as referenced herein may include volatile and non-volatile or removable and non-removable media for a storage of electronic-formatted information such as computer readable program instructions or modules of computer readable program instructions, data, etc. that may be stand-alone or as part of a computing device. Examples of computer memory may include any other medium which can be used to store the desired electronic format of information and which can be accessed by the processor or processors or at least a portion of a computing device. In various embodiments, the memory 332 may include an SD memory card, an internal and/or external hard disk, USB memory device, or similar modular memory.

In some examples, the processor 330 and the memory 332 may be referred to collectively as the transmission controller 335. The transmission controller 335 may refer to the processor 330 executing one or more methods stored in the memory 332 that control the flow of data through the patient monitor 110. In one example, the transmission controller 335 may control the function of the transceiver 324.

Program code embodied on a computer readable medium may be transmitted using any appropriate medium, including but not limited to wireless, wireline, optical fiber cable, RF, etc., or any suitable combination of the foregoing. Computer program code for carrying out operations for aspects of the present invention may be written in any combination of one or more programming languages, including an object oriented programming language such as Java, Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a LAN or a WAN, or the connection may be made to an external computer (for example, through the Internet using an Internet Service Provider).

Upon being received at patient monitor 110, the patient data may be processed by local data processing module 326. Processing the patient data may include, for example, comparing values of the patient data with one or more threshold values stored in the memory 332. For example, patient monitor 110 may include one or more lookup tables stored in the memory 332 with the one or more threshold values. During processing of the patient data, a threshold value of the one or more threshold values may be retrieved from the one or more lookup tables and compared to a corresponding value of the patient data. For example, if a blood pressure of the patient exceeds a threshold blood pressure (e.g., retrieved from the one or more lookup tables), a health status alert may be generated. In response to the health status alert being generated, an alarm may be generated, to be displayed to a caregiver at a display of the UI 336.

In some embodiments, the patient data and/or results of any processing of the patient data (e.g., health status information) may be transmitted over the second network 361 to one or more receiving devices, such as hospital information system 350, via server 340 or directly (e.g., the patient data and/or results of any processing of the patient data may be transmitted over the second network 361 to the hospital information system 350 without being first transmitted to server 340). Server 340 may also serve the patient data and/or results to a wireless device of a remote caregiver 390, so that the remote caregiver 390 may view the patient data and/or results. Further, some or all of the processing of the patient data may be carried out at server 340. For example, under some conditions, the patient data received at patient monitor 110 from the one or more sensors 370 may be transmitted to server 340 over the second network 361 without being processed at local data processing module 326, and the patient data may be processed at a cloud data processing module 342 of server 340.

Thus, the patient monitor 110 of FIGS. 1-3 may be configured to transmit and receive data over the first network 360 and the second network 361. As explained above, the data transmission duty cycle of the patient monitor 110 may be controlled based on one or more measured transmission duty cycle measurements in order to keep the transmission duty cycle of the patient monitor 110 within regulatory limits. The patient monitor 110 may be one example of a radio transmitter device; however, other radio transmitter devices may utilize the method described herein for controlling the transmission duty cycle of the radio transmitter device. Other radio transmitter devices may include cell phones, wireless tablets, radios, and other wireless devices.

The duty cycle may be controlled by the transmission controller 335 at different layers of a transmission path. The transmission path is the path is the path of data through the radio transmitter (e.g., the patient monitor 110) and may include receiving a prompt to transmit data (e.g., a prompt from a user or an automatic prompt generated by the transmission controller 335), processing and preparing the data for transmission via one or more processors, and transmitting the data through hardware such as an antenna. The transmission path may be described according to the OSI model. Each layer of the OSI model may include a portion of the transmission path. To control the transmission duty cycle of the radio transmitter, controls may be placed on one or more layers of the OSI model. The layers of the OSI model are described below to provide a framework under which the application of duty cycle controls within a transmission path can be understood; however, the controls are attached to specific functions of a transmitter and can be understood under one or more additional network models.

Turning now to FIG. 4, a pictorial representation of an OSI model 400 is shown including seven layers. The seven layers include an application layer 402, which is the seventh layer, a presentation layer 404, which is the sixth layer, a session layer 406, which is the fifth layer, a transport layer 408, which is the fourth layer, a network layer 410, which is the third layer, a data link layer 412, which is the second layer, and a physical layer 414, which is the first layer. Each layer has its own methods, functions, and hardware associated with the layer, and the layers may interact with adjacent layers. Data sent from a transmitting device, such as the patient monitor 110, to a receiving device, such as the hospital information system 350 or the remote caregiver 390, may pass through the layers of the OSI model 400 in the following order: the application layer 402, the presentation layer 404, the session layer 406, the transport layer 408, the network layer 410, the data link layer 412, and the physical layer 414. Data received by a receiving device may pass through the layers of the OSI model 400 in the following order: the physical layer 414, the data link layer 412, the network layer 410, the transport layer 408, the session layer 406, the presentation layer 404, and the application layer 402.

The application layer 402 is where users interact with network services through applications. The application layer 402 provides protocols that enable applications/software to send and receive data over a network, such as HTTP, FTP, and SMTP. The application layer 402 and a user may interact directly with a software application that implements a component of communication between the client (e.g., patient monitor 110) and server (e.g., server 340). Once data is generated at the application layer 402, the data is passed to the presentation layer 404. The presentation layer 404 translates the data into a useable format for the network. Preparing the data may include compressing the data, encrypting the data, and/or translating the data.

The data, after processing in the presentation layer, is passed to the session layer 406, which is responsible for opening, closing and managing session links between network devices. The session layer 406 receives and sends data to and from the transport layer 408. The transport layer 408 includes a variety of protocols and methods that allow for complete and reliable transmission of data between devices by managing data segmentation, error correction, and flow control. For example, at the transport layer, the data is broken into segments or datagrams and information such as port numbers and error-checking data is added.

The data from the transport layer is passed to the network layer 410, which determines the best physical path for data to travel. The network layer handles logical addressing (e.g., IP addresses), routing, and packet forwarding between devices on different networks. An example of the network is the first network 360 and the second network 361 described with respect to FIG. 3. The segments of data generated at the transport layer are encapsulated into packets or datagrams at the network layer.

The data link layer 412 manages data transfer between adjacent network nodes. The data link layer 412 is responsible for error detection, error correction, and framing. In some examples, the data link layer also schedules the transmission of data through the physical layer. For example, the packets are further encapsulated into frames at the data link layer, and MAC address and error detection bits are added.

Actions carried out at the application layer 402, the presentation layer 404, the session layer 406, the transport layer 408, the network layer 410, and the data link layer 412 may be performed and/or controlled by a processor such as the processor 330 according to instructions stored in memory such as memory 332. However, in some examples, the functions of the data link layer 412 may be performed in the hardware of a transmitting/receiving device.

The physical layer 414 encompasses physical materials and physical connections involved in the actual transmission/data transfer. The physical layer 414 may include switches, cables, and/or electronic circuits that allow for transmission of the data passed from the data link layer, such as the transceivers 324a and 324b. A bit stream of data may be transmitted and received by transmission and reception hardware such as the transceivers. The physical layer 414 may be controlled by one or more switches, which allow the bit stream to be transmitted or the transmission to be interrupted. Therefore, at the physical layer 414, data is either transmitted or blocked.

The application layer 402, the network layer 410, the data link layer 412 and the physical layer 414 are represented schematically in FIG. 5. FIG. 5 is a schematic representation of aspects of the patient monitor 110 relevant for data reception and transmission, with a plurality of the functions of the processor 330 separated into blocks to show how the functions of the processor 330 may relate to each other. The patient monitor 110 is one example of a radio transmitter device; however other radio transmitter devices may include the same components and functions as patient monitor 110 schematically represented in FIG. 5.

FIG. 5 may include a transmission path 536 and a reception path 534. The transmission path 536 tracks the path of transmitted data from a transmission request at the application layer 402 to transmission hardware within the physical layer 414. The reception path 534 tracks the path of received data from reception hardware included in the physical layer 414 to the display of that data at the application layer 402. The transmission path 536 and the reception path 534 may comprise both the physical electrical connections used to move data and the path of data through layers of data processing such as data processing performed by the processor 330 according to instructions stored in memory 332. The transmission path 536 and the reception path 534 may pass data through the same layers of the OSI model 400, but transmitted and received data may be processed differently at each layer of the OSI model 400.

The physical layer 414 encompasses hardware components of the transceiver, which in the example shown in FIG. 5 is the second transceiver 324b, such as a radio 516, a physical transmission controller 526, an RF power detector 528, and an antenna 532. The antenna 532 may be capable of receiving or transmitting wireless signals to and from a network, which in the example shown is the second network 361. In some examples, the antenna 532 may be configured to transmit and receive RF signals. The transmission path 536 may terminate at the antenna 532 and the reception path 534 may initiate at the antenna 532. The antenna 532 may be electrically coupled to the RF power detector 528 along the transmission path 536. The RF power detector 528 may be configured to detect the power usage of the transceiver over time during transmission through the antenna 532. The power usage of the transceiver may be used to determine the transmission duty cycle of the transceiver. In some examples, the RF power detector 528 may be able to generate the data used to generate a transmission envelope plot, such as the plot described with respect to FIG. 6. In some examples, the transmission duty cycle of the transceiver may be determined according to the method shown in FIG. 7, which may be carried out according to instructions stored in memory 332 and executed by the processor 330 as part of the transmission controller 335.

The RF power detector 528 may be coupled to a physical transmission controller 526 along the transmission path 536. The physical transmission controller 526 may be a switch or similar electrical component that may be positioned in either an "ON" or "OFF" position. If the physical transmission controller 526 is positioned in an "ON" position, an RF signal is allowed to be transmitted through the physical transmission controller 526 and into the RF power detector 528. If the physical transmission controller 526 is positioned in the "OFF" position, an RF signal is blocked from being transmitted through the physical transmission controller 526 to the RF power detector 528. The physical transmission controller 526 may receive instructions from the transmission controller 335 that dictate when the physical transmission controller 526 is in the "ON" state and the "OFF" state. The physical transmission controller 526 may be coupled to the radio 516. The radio 516 may be capable of transforming digital data signals to an RF signal and vice versa. The radio 516 may have a transmitted data port 522 and a transmitted RF signal port 524 as part of the transmission path 536 and a receiving data port 518, and a receiving RF signal port 520 as part of the reception path 534. The transmitted data port 522 may receive digital data from the network layer 410. The digital data may then be transformed by the radio 516 into an RF signal that is emitted at the transmitted RF signal port 524 and transferred to the physical transmission controller 526 as part of the transmission path 536. The receiving RF signal port 520 may receive an RF signal from the antenna 532. The RF signal is then transformed within the radio 516 into a digital signal that is output at the receiving data port 518 as part of the reception path 534.

The radio 516 may provide some of the functions of the data link layer 412 under the directions of the transmission controller 335. In some examples, the radio 516 may process data for transmission by dividing it into a transmittable form. For example, the radio 516 may encapsulate packets of digital data received at the transmitted data port 522 into frames of data. The radio 516 may convert data into a stream of bits that is transmitted through the transmitted RF signal port 524. Similarly, the radio 516 may assemble a stream of bits received at the receiving RF signal port 520 into data that may be output at the receiving data port 518. In some examples, transmission of data through the data link layer may be allowed or blocked at the radio 516 (e.g., in response to commands from the transmission controller 335). Additionally, the retransmission of data through the data link layer may be blocked or allowed at the radio 516 in response to directions from the transmission controller 335. Transmitted digital signals received at the transmitted data port 522 may be received from a data rate limiter 514 that performs traffic shaping within the network layer 410. The functions performed by the network layer 410 may be executed by the transmission controller 335. As described with respect to FIG. 4, the network layer 410 may be responsible for routing. The network layer 410 may perform routing on both data transmitted from the network layer 410 to the transmitted data port 522 along the transmission path 536 and data received at the network layer 410 from the receiving data port 518 along the reception path 534. The network layer 410 may also be responsible for traffic shaping. Traffic shaping is a method for managing the rate at which data flows out of the network layer along the transmission path 536 that includes storing data in a short term storage and selectively managing which data is released from storage and the rate at which the selected data is released from storage to manage traffic through the network. The data rate limiter 514 may be responsible for controlling the rate at which data is released from short term storage. The data rate limiter 514 may receive instructions from the transmission controller 335 that instruct the data rate limiter 514 to restrict the rate of data flow, maintain the rate of data flow, or increase the rate of data flow through the network layer 410 in response to changes in the measured transmission duty cycle. For example, if the transmission duty cycle is too high, the transmission controller 335 may instruct the data rate limiter 514 to lower the rate at which data is released from short term storage. In another example, if the transmission duty cycle is low, the transmission controller 335 may instruct the data rate limiter 514 to increase the rate at which data is released from short term storage. In some examples, the data rate limiter 514 may impose a maximum data rate (in bps) on traffic through the network layer 410.

The network layer 410 is in communication with one or more intermediate layers 538. The intermediate layers 538 include the transport layer 408, the session layer 406, and the presentation layer 404. Like the network layer 410, the transport layer 408, the session layer 406, and the presentation layer 404 each have a role in facilitating communication between devices, and the role of each layer may be performed by the transmission controller 335. The functions, methods, and hardware associated with each layer as described with respect to FIG. 4 are applied to data processed along the transmission path 536 and data processed along the reception path 534. Data input to the intermediate layers 538 along the transmission path 536 is input from the application layer 402 and be output by the intermediate layers 538 to the network layer 410. Data input to the intermediate layers 538 along the reception path 534 is input from the network layer 410. Data output from the intermediate layers along the reception path 534 may be output in a form useable for the application layer 402. In some examples, the intermediate layers 538 may not be involved in the control of the transmission duty cycle of the patient monitor 110 and may not receive instructions from the transmission controller 335 related to transmission duty cycle control. In such examples, one data is released from the application layer 402, the data may pass through the intermediate layers as described above with respect to FIG. 4, without adjustment or control based on duty cycle. However, in other examples, one or more of the intermediate layers may be controlled based on the transmission duty cycle.

The application layer 402 is in communication with the intermediate layers 538. Like the network layer 410 and the intermediate layers 538, the application layer 402 includes one or more functions used in the communication between devices, and the functions of the application layer 402 may be performed by the transmission controller 335. The application layer 402 may include and/or interface with application software 508 and a data control module 510. The application software 508 may receive data from the intermediate layers 538 at a data input port 540 and output data through a data output port 542. The application software 508 may be software configured to support the UI 336, process received patient monitoring data, and/or perform other functions. For example, the application software 508 may allow the UI 336 to display one or more patient health metrics collected from the sensors 370 from the patient monitor 110, as well as select which health metrics are collected and displayed. In some examples, the UI 336 of the patient monitor 110 may allow the patient 102 to interact with the clinician 106, such as by allowing the patient 102 to request assistance from the clinician 106. In some examples, the application software 508 may determine what patient monitoring data is to be sent to the server 340 and when. Along the reception path 534, the application software 508 may receive transmitted requests from the second network 361 that pass through the physical layer 414, the data link layer 412, the network layer 410, and the intermediate layers 538 before being received at the data input port 540 of the application software 508. The application software 508 may process the request from the second network 361, and initiate the transfer of data along the transmission path 536 in response. For example, the application software 508 may process a request received through the second network 361 to begin transmitting a particular health metric/patient monitoring data, such as the heart rate of the patient 102. Transmission of data may then be initiated at the application software 508 and proceed through the data output port 542 along the transmission path 536. The application software 508 may be capable of receiving data from the sensors 370. In some examples, the transmission path 536 may be utilized when the application software 508 processes requests made through the UI 336 and converts those requests to transmittable data. In some examples, the application software 508 may generate transmission requests internally, such as generating a request to upload data according to a timed schedule, and initiate the transmission of data along the transmission path 536 without input from the UI 336 or the second network 361. In some examples, the transmission path 536 may be utilized to transmit healthcare data (e.g., the health metrics/patient monitoring data mentioned above), such as heart rate, pulse oximetry, and respiratory rate.

The transmission path 536 may include a data control module 510. The data control module 510 may be capable of limiting the rate of data flow out of the application layer 402 along the transmission path 536. In some examples, the data control module 510 may utilize multiple queues with different priorities to control the rate of data flow out of the application layer. The data control module 510 may receive instructions from the transmission controller 335 to increase or decrease data flow through the application layer 402. The queues may be ranked based on the priority level of each queue, with higher priority data assigned to higher priority queues and lower priority data assigned to lower priority queues. If data flow through the application layer 402 is reduced, data flow may be reduced through the lower priority queues first, to allow data to flow through the higher priority queues unimpeded. In some examples, certain types of data traffic may be blocked by the data control module 510 to reduce traffic through the application layer 402. Examples of blocked data types may include software updates, backfill data, and/or waveforms.

In some examples, the patient monitor 110 may be communicatively coupled to sensors 370, which may include the sensor 208 that is capable of measuring the respiration rate of the patient 102. The sensor 208 may be able to report the respiration rate of the patient 102 as a single number, but the sensor 208 may further be able to collect and report a real time plot of the respiratory activity of the patient 102 (or alternatively, the patient monitor 110 may be configured to store respiration rate values received from the sensor 208 over time and assemble a plot of the respiration activity of the patient 102). The sensors 370 may further include a camera capable of collecting real-time video of the patient 102. The highest-priority data for the clinician 106 to receive may be the respiration rate of the patient 102 so that the clinician 106 can know if patient respiration is stable or if the patient respiration is changing. Higher priority data may be assigned to higher priority queues that are unlikely to be affected by measures to reduce the duty cycle of the transmitter. Data assigned to lower priority queues may include live video streams and live streams of plots such as plots of respiratory activity or a live streamed ECG. The priority of each kind of data may depend on the patient, and in some cases, a clinician or administrator may adjust the hierarchy of priority of types of data by interacting with the UI 336 or by utilizing a device connected to the patient monitor 110 through the second network 361.

In examples where the transmitting device is not the patient monitor 110, the data may not be physiological patient data, and may be sorted into queues according to the priorities of the device and associated network. For example, a cell phone may sort location data and text communication in higher priority queues than transmitting a live stream of video from the cell phone.

In the event that the transmission controller 335 instructs the data control module 510 to reduce data flow through the application layer 402, the data control module 510 may stop the transmission of data in lower priority queues or reduce the quality of data transmitted from lower priority queues. In some examples, the quality of data in lower priority queues may be reduced according to instructions from the transmission controller 335. For example, the resolution of a video feed being transmitted by the patient monitor 110 may be reduced, or the amount of data points on a live streamed plot being transmitted per second may be reduced. According to the methods described above, data flow through the application layer 402 can be reduced by limiting or halting data flow from lower priority queues while data flow from higher priority queues is unimpeded.

The transmission path 536 includes controls on the amount of data being transmitted at the data control module 510 in the application layer 402, the data rate limiter 514, the radio 516, and the physical transmission controller 526. The controls on the amount of data being transmitted are all controlled by the transmission controller 335 in response to measurements of the transmission duty cycle of the transmitter device measured over one or more time periods by the RF power detector 528.

FIG. 6 depicts a plot 600 of the transmission envelope of a radio transmitter included in a radio transmitter device, such as the patient monitor 110. Time is represented along an x-axis 602. The present moment 616 is the time at which the transmitter status (on/off) is measured, the portion of the x-axis 602 to the right of the present moment 616 is the future and the portion of the x-axis 602 to the left of the present moment 616 is the past. The past includes the transmission envelope 614 of the radio transmitter. The x-axis 602 includes a first interruption 610 and a second interruption 612. The first interruption 610 and the second interruption 612 each represent a period of time for which the transmission envelope 614 has been recorded but is not included in FIG. 6 for visual clarity. The transmission envelope 614 has a height along a y-axis 604 that represents the amplitude of the transmission envelope 614. The transmitter is either in the "ON" state, represented by a first amplitude 606, or the "OFF" state, represented a second amplitude 608. When the transmission envelope 614 has an amplitude equal to the first amplitude 606, the transmitter is actively transmitting data. When the transmission envelope has an amplitude equal to the second amplitude 608, the transmitter is not transmitting data. The transmission duty cycle of the transmitter may be calculated by recording the portion of time the transmitter is actively transmitting during a given time period (e.g., by recoding samples of the transmit signal state or with an integrated circuit that is configured to perform integration). The amount of time the transmitter is actively transmitting may be calculated by integrating the amount of time the transmission envelope 614 is equal to the first amplitude 606 over a given time period. The duty cycle may then be determined by dividing the amount of time the transmitter is transmitting by the given time period. For example, the duty cycle may be calculated according to the equation duty cycle=cumulative_TX_on_time/integration_time. Transmission on time (e.g., TX_on_time) integration may be performed by using software or dedicated hardware/integrated circuit. Integration and calculation may be computationally efficient as the TX_on pulses are relatively long (typically > 100 us). The duty-cycle calculation interval depends on the integration time (e.g., for a 10% of the integration time, if int_time 0.1s, the duty cycle value is recalculated every 10ms).

The plot 600 includes three example time periods over which the transmission duty cycle is calculated. In other embodiments, the transmission duty cycle may be calculated over any number of time periods. For example, the transmission duty cycle may be calculated over less than three time periods or more than three time periods. The three time periods include a first time period 618, a second time period 620, and a third time period 622. The first time period 618 may be the shortest time period. In the illustrated example, the first time period 618 may be 0.1 s before the present moment 616. Calculating the duty cycle of the transmitter over the first time period 618 may identify rapid changes in the duty cycle of the transmitter, such as sudden spikes in the duty cycle. The second time period 620 may be an intermediate time period, and in the illustrated example may be 1 s before the present moment 616. Calculating the duty cycle of the transmitter over the second time period 620 may identify changes in the duty cycle of a transmitter over an intermediate time scale. The third time period 622 may be the longest time period, and in the illustrated example may be 300 s before the present moment 616. Calculating the duty cycle of the transmitter over the third time period 622 may identify changes in the duty cycle of a transmitter over a long term scale, such as a sustained increase in duty cycle. Three time periods are shown in the plot 600, however in some examples more than three or less than three time periods may be monitored. Further, the time periods may have different durations without departing from the scope of this disclosure (e.g., the first time period may be 0.2s; the second time period may be 1.5s; the third time period may be 250s, etc.).

The measured duty cycle over one or more or each of the time periods may be used by the transmission controller 335 to determine whether to restrict, increase, or maintain data flow through one or more layers of the OSI model 400. For example, the physical layer 414 and the data link layer 412 may be controlled based on the measured duty cycle over the first time period 618 and the measured duty cycle over the third time period 622, the network layer 410 may be controlled based on the measured duty cycle over the second time period 620, and/or the application layer 402 may be controlled based on the measured duty cycle over the third time period 622.

The measured duty cycle over the third time period 622 may be used by the transmission controller 335 to control the data control module 510 within the application layer 402 (and specifically control the flow of data out of the application layer). The data control module 510 may be capable of limiting the flow of data, and therefore the transmission duty cycle, with the smallest interruption to the flow of transmitted data through the radio transmitter device relative to the interruptions to the flow of transmitted data caused by data flow controls imposed on the network layer 410, the physical layer 414 and the data link layer 412. However, in some examples, the data control module 510 may not be able to reduce the flow of data on short timescales, such as timescales below 1 s. For example, because data that flows out of the application layer travels through/is controlled by downstream layers (e.g., the network layer), a change in the amount of data output by the application layer does not result in an immediate change to the transmission duty cycle. As a result, the data control module 510 may be used to control the flow of data on longer timescales, such as the third time period 622. In some examples, the duty cycle measured over the third time period 622 may also be used to control the physical transmission controller 526 in order to control data transmission through the physical layer 414 and the data link layer 412. Regulatory limits, such as regulatory limits based on SAR, may be calculated on a timescale equal to or comparable to the third time period 622. If the measured duty cycle over the third time period 622 exceeds a set threshold configured to be less than a regulatory limit, the transmission controller 335 may provide instructions to block data transmission through the physical layer 414 or the data link layer 412. The physical transmission controller 526 may be able to block transmission of data within a timespan on the millisecond scale. Immediately blocking the transmission of data when the duty cycle measured over the third time period 622 exceeds the set threshold may prevent the duty cycle from exceeding regulatory limits. Blocking transmission using the physical transmission controller 526 in addition to the data control module 510 ensures that the transmitter operates within regulatory limits.

The measured duty cycle over the second time period 620 may be used by the transmission controller 335 to control the data rate limiter 514 to control the data rate through the network layer 410. Decreasing the transmission duty cycle using the data rate limiter 514 may result in delays in data transmission, which may be noticeable for the user in some examples. However, an advantage of utilizing the data rate limiter 514 is that there is limited latency in enacting duty cycle controls while using the data rate limiter 514. For example, controlling the data rate (e.g., reducing the data rate) using the data rate limiter 514 may decrease the measured duty cycle of the radio transmitter within the second time period 620. Therefore, control of the data rate limiter 514 may be based on the second time period 620.

The measured duty cycle over the first time period 618 may be used by the physical transmission controller 526 to control data transmission through the physical layer 414 and the data link layer 412. The physical transmission controller 526 is only configured to allow transmission or block transmission. Blocked transmissions may cause significant interruptions in the transmission of data over long periods of time. However, the physical transmission controller 526 may be able to respond to commands to block or allow transmission within a relatively short time frame. In some examples, the physical transmission controller 526 may be able to respond to commands on a millisecond time scale. Because of the short response time of the physical transmission controller 526, the physical transmission controller 526 may be used to reduce the duty cycle of the device in response to an increased duty cycle measured over a short time period, such as the first time period 618. Data flow reductions at the application layer 402 and the network layer 410 in response to increased duty cycles measured over longer time periods may constrain the use of the physical transmission controller 526 to blocking sudden sharp increases in the duty cycle of the transmitter, which may minimize the interruptions caused by blocking transmission at the physical layer 414 and the data link layer 412.

FIG. 7 illustrates a method 700 to control a duty cycle of a radio transmitter based on the measured transmission duty cycle over one or more time periods. The method 700 further includes using the measured transmission duty cycles to determine how the flow of data through various layers of the OSI model, such as the application layer 402, the network layer 410, the data link layer 412, and the physical layer 414, is to be controlled to ensure the transmission duty cycle of the transmitter remains within regulatory or operational limits. The method 700 may be executed by a radio transmitter device, such as patient monitor 110, that includes a radio transmitter, such as transceiver 324, according to instructions stored in memory and executed by one or more processors, such as the memory 332 and processor 330 of the transmission controller 335.

At 702, the method 700 includes receiving data from an RF power detector, such as RF power detector 528. The RF power detector may be capable of detecting and recording the transmission envelope of a transmitted signal. The transmission envelope 614 described with respect to FIG. 6 is one example of a transmission envelope that may be received by the transmission controller at 702. At 704, the method 700 may include integrating the time the radio transmitter is actively transmitting over a plurality of time periods. Integrating over the duration the radio transmitter is actively transmitting for each of the plurality of time periods may include integrating the amount of time within each of the time periods the transmission envelope has an amplitude equal to a first amplitude, which indicates the radio transmitter is actively transmitting. The integration process may be performed over each of a plurality of time periods such as the first time period 618, the second time period 620, and the third time period 622 of FIG. 6 to produce an integrated transmission time for each time period. If at 704 the radio transmitter and the RF power detector have not been powered on for a portion of one or more time periods, any time before the transmitter is powered on is factored into the measured duration of active transmission as an amount of time the transmitter is not actively translating. For example, the moment the radio transmitter is powered on, the active transmission time for each time period is 0 s and the transmitter duty cycle is 0%. If it is uncertain if the radio transmitter is actively transmitting for a duration, the duration of the uncertainty is assumed to be 100% duty cycle and included within the active transmission time of the radio transmitter. The integrated transmission times may be used at 706 to calculate the transmission duty cycle of the transmitter over each of the plurality of time periods. Thus, one or more duty cycles may be calculated, such as a first duty cycle D1 calculated over the first time period, a second duty cycle D2 calculated over the second time period, and a third duty cycle D3 calculated over the third time period, where the second time period is longer than the first time period and the third time period is longer than the second time period. The transmission duty cycle is the fraction of the time period during which the radio transmitter is transmitting. The transmission duty cycle over each time period may be calculated by dividing the integrated transmission time for the time period by the time period. For example, referring back to FIG. 6, the first time period 618 may be 0.1s and integrating the waveform where the amplitude is equal to the first amplitude may result in an integrated transmission time of 0.05s. Dividing the integrated transmission time (of 0.05s) by the time period (0.1s) may result in a duty cycle of 0.5 (which may also be represented as 50%).

At 708, the method 700 may include adjusting the flow of data through one or more layers of a transmitter model based on the transmission duty cycle measured over each time period. The transmitter model may be the OSI model 400, which includes the application layer 402, the network layer 410, the physical layer 414 and the data link layer 412, among other layers. Adjusting the data flow through the one or more layers may include, at 710, assessing the first transmission duty cycle D1 and the third transmission duty cycle D3 measured over a first time period and third time period, respectively, such as the first time period 618 and the third time period 622, and adjusting the physical layer and the data link layer in response, which is explained in more detail below with respect to a method 800 of FIG. 8. Adjusting the data flow through the one or more layers may include, at 712, assessing the second transmission duty cycle D2 measured over a second time period, such as the second time period 620, and adjusting the network layer based on the second duty cycle D2, which is explained in more detail below with respect to a method 900 of FIG. 9. Adjusting the data flow through the one or more layers may further include, at 714, assessing the third transmission duty cycle D3 measured over the third time period, such as the third time period 622, and adjusting the application layer based on the third duty cycle D3, which is explained in more detail below with respect to a method 1000 of FIG. 10. As described, method 700 includes adjusting the flow of data according to duty cycles calculated over three time periods. However, in some examples, the duty cycle of the radio transmitter may be calculated over more than three time periods and the duty cycles calculated over the additional time periods may be used to control the flow of data through the radio transmitter by controlling the data flow through one or more layers based on the calculated duty cycle(s) relative to one or more thresholds. In other examples, the duty cycle may be calculated over fewer than three time periods, such as only two time periods. For example, the duty cycle over the third time period and the duty cycle over the first time period may be determined and used to control the flow of data through the radio transmitter, and the duty cycle over the second duty cycle may not be measured. Calculating the duty cycle over fewer time periods may reduce the computational demands on the transmission controller, but not adjusting the flow of data based on the second duty cycle may increase interference between collocated transmitters. In other examples, the duty cycle may be calculated over only the second time period and the third time period, or the duty cycle may be calculated over only the third time period. Thus, the decision of which time periods over which to calculate the duty cycle and the associated controls based on the calculated duty cycles may be based on which metrics are prioritized, e.g., whether or not it is a priority to avoid interference, while ensuring regulatory limits are met.

The method 700 may be performed at a suitable frequency. In some examples, the method 700 may be performed substantially continuously, such that transmission duty cycle measurements are calculated at the same, higher frequency (e.g., once every 0.1s) and the transmission controller 335 updates control instructions to the application layer 402, the network layer 410, the data link layer 412, and the physical layer 414 at the higher frequency. In such examples, the time periods over which the respective duty cycles are measured may be sliding windows (e.g., windows that slide every 0.1s or other frequency). The various duty cycles may be calculated even before the associated time period has fully elapsed. For example, the third time period is relatively long, such as 300s. Waiting to calculate the duty cycle over the third time period until 300s after the transmitter has been powered on may result in excessive controls at the data link or physical layer, owing to the lack of controls occurring at the application layer. Thus, each duty cycle may be measured at the set frequency starting from when the transmitter is powered on, with the time period before the transmitter is powered on set to 0%. In this case, the duty cycle over the third time period may be 0% at the time the transmitter is powered on, and then may increase as the transmitter is used and transmitter on times are detected and included in the measured duty cycle. After the third time period since the transmitter was powered on has elapsed, the duty cycle measured over the third time period may reflect only the time that the transmitter was powered on. In other examples, at least some aspects of the method 700 may be performed at a lower frequency, e.g., intermittently. In some examples, the different transmission duty cycles may be calculated at different frequencies. For example, the third transmission duty cycle may be measured over the third time period 622, which in some examples may be 3000 times longer than the first time period 618. In some examples, the third transmission duty cycle may be calculated less frequently than the first transmission duty cycle. For example, the first transmission duty cycle may be calculated every 0.1s while the third transmission duty cycle may be calculated every 300 s. In some examples, the method 700 may include adjusting the flow of data through layers of the transmitter model each time a relevant transmission duty cycle measurement is recalculated. In the example that the first transmission duty cycle is calculated more frequently than the third transmission duty cycle, the method 1000 may be performed at 714 each time the third transmission duty cycle is calculated and the method 800 may be performed at 710 each time the first transmission duty cycle is calculated. Thus, the method 1000 may be performed less frequently than the method 800. It is to be appreciated that the time periods are independent and can be calculated at the same frequency or at different frequencies without departing from the scope of this disclosure.

Turning now to FIG. 8, FIG. 8 includes the method 800 for controlling the flow of data through the physical layer and the data link layer. The method 800 may be executed by a radio transmitter device, such as patient monitor 110, that includes a radio transmitter, such as the transceiver 324, according to instructions stored in memory and executed by one or more processors, such as the memory 332 and the processor 330 of the transmission controller 335. In some examples, method 800 may be carried out as part of method 700. For example, method 800 may be carried out at 710 of method 700.

The physical layer and the data link layer may include a physical transmission controller, such as the physical transmission controller 526 of FIG. 5. The physical transmission controller may be capable of blocking or allowing transmission of data through the physical layer and the data link layer. The method 800 includes instructions for triggering the physical transmission controller to block or unblock transmission based on two measured duty cycles.

At 802, the method 800 may include obtaining a first transmitter duty cycle D1 and a third transmitter duty cycle D3. The transmitter duty cycles may be calculated as part of the method 700, as explained above. The first duty cycle D1 may be calculated across a first time period, such as the first time period 618. The third duty cycle D3 may be calculated across a third time period, such as the third time period 622. The third time period may be significantly longer than the first time period and in one example may be at least 1000 times longer than the first time period.

At 804, the method 800 may include determining if the third duty cycle D3 is greater than a third threshold duty cycle. The third threshold duty cycle may be a positive non-zero value between 0% and 100% transmission measured over a nonzero time period. The third threshold duty cycle may be established such that the transmitter would be in compliance with regulatory limits for the SAR of people in contact with the radio transmitter if the radio transmitter were to operate at or below the third threshold for a duration such as the third time period. In some examples, the third threshold is set below regulatory limits. The third time period may be based on regulatory limits, such as the standard time scale for measuring the duty cycle of a radio transmitter when calculating SAR. In one example, the third threshold may be a transmission duty cycle of 10% over 300 s. If the third transmitter duty cycle is greater than the third threshold at 804, the method 800 may proceed to 806, where the method 800 may include instructing the physical transmission controller to block transmission through the physical layer and the data link layer. In the example that the physical transmission controller is a switch, the switch may be instructed to open to block transmission. In some examples, the switch may be a physical (e.g., hardware) switch, while in other examples, the switch may be a logical (e.g., software) switch. Blocking transmission reduces the duty cycle of the transmitter by preventing transmission for an amount of time, which prevents the transmitter from exceeding regulatory limits on the radio transmitter device with no latency. Blocking the duty cycle of the transmitter at the physical level guarantees that the transmitter does not exceed regulatory limits, which may allow the third threshold duty cycle to be considered the maximum duty cycle of the transmitter when the radio transmitter device is assessed for regulatory compliance. This may allow the radio transmitter device to be designed with more flexibility. In some examples, when data transmission is blocked through the physical layers or data link layers, the data may be stored in short term memory, such as the memory 332 until data transmission is allowed. In some examples, the default state of the switch may be open, and thus blocking transmission may include maintaining the switch in the open position, if already open, and may not include sending a command to the switch to open. If the third transmitter duty cycle D3 is less than the third threshold at 804, then the transmitter may be operating within regulatory limits over the third time period and the method may proceed to 808, where transmission is allowed at the physical layer and the data link layer. Allowing transmission may include sending an instruction from the transmission controller to the physical transmission controller to allow transmission. In the example that the physical transmission controller is a switch, the switch may be instructed to close or remain closed to allow transmission.

At 810, the method 800 may include determining if the first transmitter duty cycle D1 is greater than a first threshold. The first threshold may be a positive nonzero value between 0% and 100% measured over a nonzero time period that may be equal to the first time period. The acceptable duty-cycle over some specific time (e.g., the first threshold) depends on the radio transmitter device and the system in which the radio transmitter device operates. For example, in the case of a specific radio operating on the MBAN (e.g., the patient monitor disclosed herein), the first threshold for the transmission duty cycle may be 30% over any 0.1s time. The first threshold may be set to enable in-device radio coexistence. In a device with multiple radios, a radio transmitter transmitting at a first frequency band generates wideband noise that blocks other receivers within the device from receiving within the first frequency band. To enable in-device radio coexistence, the duty cycle of a radio transmitter producing interference may be controlled to a duty cycle below the first threshold, measured under a short period of time, such as the first time period 618. Transmission through the physical layer is adjusted in response to the first measured duty cycle D1 exceeding the first threshold because the physical transmission controller is capable of switching between allowing transmission and blocking transmission within milliseconds. If the first duty cycle D1 is greater than the first threshold at 810, the method 800 proceeds to 812, where the method 800 may include blocking transmission through the physical layer and the data link layer by sending an instruction from the transmission controller to the physical transmission controller to allow transmission. Blocking transmission through the physical layer and the data link layer may lower the duty cycle of the transmitter within the first time period and allow for in-device radio coexistence. If the first duty cycle D1 is less than the first threshold at 810, then the transmitter is operating in such a way to allow for in-device radio coexistence and the method 800 may proceed to 814, where the method 800 includes allowing transmission through the physical layer and the data link layer by sending an instruction from the transmission controller to the physical transmission controller to allow transmission.

The first threshold may be a larger duty cycle over a shorter time period compared to the third threshold because the third threshold is established in compliance with regulatory limits on SAR. To meet regulatory limits on SAR, the transmitter duty cycle may be low over a relatively long period of time. In contrast, the first threshold is established to ensure the transmitter device is capable of in-device transmitter coexistence. To enable in-device transmitter coexistence, the transmitter duty cycle may remain under a large duty cycle threshold over a short period of time. In one example, the first threshold may be established to prevent sudden spikes in the transmitter duty cycle.

Turning now to FIG. 9, FIG. 9 includes the method 900 for controlling the flow of data through the network layer. The method 900 may be executed by a radio transmitter device, such as the patient monitor 110, that includes a radio transmitter, such as the transceiver 324, according to instructions stored in memory and executed by one or more processors, such as the memory 332 and the processor 330 of the transmission controller 335. In some examples, method 900 may be carried out at 712 of method 700. The network layer may include a data rate limiter, such as the data rate limiter 514. The data rate limiter may control the rate at which data leaves the network layer and is transferred to the data link layer. The data rate limiter may be controlled by the transmission controller.

At 902, the method 900 may include obtaining a second transmitter duty cycle D2. In some examples, the second transmitter duty cycle D2 may be calculated as part of the method 700, explained above. The second transmitter duty cycle D2 may be calculated over a second time period such as the second time period 620. At 904, the method 900 may include determining if the second transmitter duty cycle D2 is greater than a second threshold range. The second transmitter duty cycle D2 may be greater than the second threshold range if the second transmitter duty cycle D2 is greater than a second upper threshold of the second threshold range. The second threshold range may be a range of duty cycles calculated over the second time period that may be defined by the second upper threshold and a second lower threshold. In some examples, the second threshold range may be established to enable multiple transmitter devices to coexist on the same channel without interfering with each other and without exceeding regulatory limits for transmission. In one example, multiple patient monitors similar to patient monitor 110 may be in use in the same patient monitoring environment 100. To allow each patient monitor to transmit within the patient monitoring environment with minimal interference, each patient monitor may be controlled to maintain a transmitter duty cycle within the second threshold range. The second threshold range may depend on the number of radio transmitter devices operating in close proximity to each other and transmitting on the same channel. In the example that five patient monitors are in use on the same channel, the second threshold range may be transmission duty cycle values between a second lower threshold of 15% measured over 1 s and a second upper threshold of 20% measured over 1 s. The data rate limiter may be able to adjust the transmission duty cycle of the radio transmitter with a few seconds of latency. In some examples, the second threshold range may be set such that the changes in transmission duty cycle of the transmitter device during the latency period of the data rate limiter do not significantly reduce system performance or exceed regulatory limits.

At 904, the method 900 may include determining if the second duty cycle D2 is greater than the second threshold range. The second duty cycle D2 is greater than the second threshold range if the second duty cycle D2 is greater than the largest duty cycle included within the second threshold range (e.g., greater than the second upper threshold described above). In one example, for a system of five patient monitors transmitting over a local network, the second upper threshold is 20% measured over 1 s. If the second duty cycle D2 is greater than the second threshold range, the method 900 may proceed to 906. At 906, the method 900 includes decreasing the data rate through the network layer. Decreasing the data rate at 906 may allow the transmitter device to coexist with other transmitter devices sharing the same channel. In some examples the data rate limiter may perform traffic shaping to reduce the data rate through the network layer. Traffic shaping may include storing data that is set to be transmitted in short term memory, and controlling the rate at which the data is released from short term memory. In some examples, the data rate may be decreased by a percentage of the maximum data rate. In other examples, the data rate may be reduced by a set amount, such as reducing the data rate by a specific number of bits per second. In another example, the data rate may be reduced by an amount proportional to the amount by which the second duty cycle D2 exceeds the second threshold range. For example, the data rate may be reduced by 10% for every 5% the second duty cycle D2 is over the second threshold range. If the second duty cycle D2 is not greater than the second threshold range at 904, the method 900 may proceed to 908. At 908, the method 900 may include not changing the data rate through the network layer. The data rate limiter may release data at the same rate the data rate limiter had been when the method 900 was initiated. Thus, a data rate through the network layer that results in a transmitter duty cycle within the threshold range may be preserved.

At 910, the method 900 may include determining if the second duty cycle D2 is less than the second threshold range. The second duty cycle D2 is less than the second threshold range if the second duty cycle D2 is less than the smallest duty cycle included within the second threshold range (e.g., the second lower threshold described above). In one example, second lower threshold is 15% measured over 1 s. If the second duty cycle D2 is less than the second threshold range, the method 900 may proceed to 912, where the method 900 may include increasing the data rate through the network layer. Increasing the data rate may allow the transmitter to operate efficiently, reduce delays in the transmission of data and reduce the risk of overflowing the short term memory where data is temporary stored within the data rate limiter. Traffic shaping may be employed by the data rate limiter to increase the rate at which data is transferred out of the network layer. Similar to the plurality of methods possible for decreasing the data rate, there are multiple methods for increasing the data rate. In some examples, the data rate may be increased by a percentage of the maximum data rate. In other examples, the data rate may be increased by a set amount, such as increasing the data rate by a specific number of bits per second. In still further examples, the data rate may be increased by an amount proportional to the amount by which the second duty cycle D2 is less the second threshold range. For example, the data rate may be increased by 10% for every 5% the second duty cycle D2 is under the second threshold range. If the second duty cycle D2 is not less than the second threshold range at 910, the method 900 may proceed to 914, where the method 900 may include not changing the data rate through the network layer. Thus, the data rate is unchanged if the second duty cycle is within the second threshold range, the data rate is increased if the second duty cycle D2 is below the second threshold range, and the data rate is decreased if the second duty cycle D2 is greater than the second threshold range.

Turning now to FIG. 10, FIG. 10 includes the method 1000 for controlling the flow of data through the application layer. The method 1000 may be executed by a radio transmitter device, such as the patient monitor 110, that includes a radio transmitter, such as the transceiver 324, according to instructions stored in memory and executed by one or more processors, such as the memory 332 and the processor 330 of the transmission controller 335. In some examples, method 1000 may be carried out at 714 of method 700. The application layer may include a data control module, such as the data control module 510. The data control module may control the flow of transmitted data out of the application layer. In some examples, the data control module may control the flow of transmitted data by sorting data into queues with different priority levels, then selectively transmitting data from the queues depending on the priority level of the queue that contains the data. Using queues to control the flow of data may provide limited interruptions to the flow of high-priority data while reducing the duty cycle of the transmitter by limiting the transmission of lower priority data. However, data control at the application layer may include a significant amount of latency between the initiation of data control at the application layer and an impact on the duty cycle of the device because the transmitted data is processed by each layer of the OSI model before the transmitted data is transmitted and the duty cycle is measured. Thus, data rate limitations on the application layer may be used in conjunction with controls on the network layer and the physical layer to keep the duty cycle of the transmitter within regulatory limits.

At 1002, the method 1000 may include obtaining a third transmitter duty cycle D3. In some examples, the third transmitter duty cycle D3 may be calculated as part of the method 700, explained above. The second transmitter duty cycle D3 may be calculated over a third time period such as the third time period 622. In one example, the third time period is 300 s. At 1004, the method 1000 may include determining if the third transmitter duty cycle D3 is greater than a fourth threshold range. To be greater than the fourth threshold range, the third transmitter duty cycle D3 may be greater than the maximum value of the fourth threshold range. The fourth threshold range may be established such that the maximum value of the fourth threshold range is less than the third threshold described with respect to the method 800. By establishing that the maximum duty cycle of the fourth threshold range is less than the third threshold, duty cycle limitations may be imposed at the application level before duty cycle limitations are imposed at the physical layer. The transmission duty cycle may be less likely to exceed the third threshold if the duty cycle is limited when the duty cycle exceeds the maximum duty cycle included within the fourth threshold range. In one example, the third threshold may be 10% over 300 s and the maximum duty cycle within the fourth threshold range may be 8% over 300 s. Using the lower threshold value (8% vs. 10%) at application level guides the controls to occur at the application level, as opposed to the physical level.

If the third transmitter duty cycle D3 is greater than the fourth threshold range at 1004, the method 1000 may proceed to 1006. At 1006, the method 1000 may include decreasing the data rate through the application layer by utilizing priority-ranked queues. In one example, the process for decreasing the data rate may depend on the degree to which the third transmission duty cycle D3 exceeds the fourth threshold range. In one example, if the third transmission duty cycle D3 exceeds the fourth threshold by less than 1%, the data control module may reduce the flow of data through the application layer less aggressively than if the third transmission duty cycle exceeds the fourth threshold by more than 1 %. In the example that the fourth threshold is 8% over 300 s, more aggressive data control methods may be used if the third transmission duty cycle D3 is greater than 9% and less aggressive data control methods may be used if the third transmission duty cycle D3 is between 8% and 9%.

Less aggressive methods to reduce the flow of data through the application layer may include reducing the frame rate of transmitted videos, reducing the sampling rate being transmitted for live data streams, and/or stopping the transmission of data within one or more of the lower priority queues. Less aggressive methods to reduce the flow of data may preserve the transmission of data through the higher priority queues and one or more lower-priority queues, but may not reduce the duty cycle of the transmitter as much as more aggressive methods of limiting the transmission of data through the application layer. More aggressive methods of reducing the flow of data through the application layer may include blocking the transmission of data through one or more lower priority queues, as well as one or more queues that have a moderate priority level. Additionally, queues that include large amounts of data, such as videos, may be blocked or the rate of transmission within those queues may be reduced. More aggressive methods to reduce the flow of data through the application layer may reduce the transmission duty cycle of the transmitter more than the less aggressive methods to reduce the flow of data through the application layer, but may have a more significant impact on the amount of data being transmitted from the application layer.

If the third transmission duty cycle is not greater than the fourth threshold range at 1004, the method 1000 may proceed to 1008. At 1008, the flow of data through the application layer is not changed. In one example, not changing the flow of data through the application layer may include transmitting data from the same queues that were transmitting data when the third transmitter duty cycle D3 was obtained at 1002.

At 1010, the method 1000 may include determining if the third transmitter duty cycle D3 is less than the fourth threshold range. In one example, the fourth threshold range may be duty cycles between 7% and 8% measured over 300 s, the minimum duty cycle of the fourth threshold range may be 7% and the maximum duty cycle of the fourth threshold range is 8%. To be less than the fourth threshold range, the third transmitter duty cycle D3 may be less than the minimum duty cycle of the fourth threshold range. If the third transmitter duty cycle D3 is less than the fourth threshold range at 1010, the method may proceed to 1014. At 1014, the method may include increasing data flow through the application layer by utilizing priority ranked queues. In some examples, the data flow through the application layer may be increased proportionally to the difference between the third transmitter duty cycle D3 and the minimum duty cycle of the fourth threshold range. In one example, more aggressive methods to increase the flow of data through the application layer may be used if the third transmission duty cycle is less than the fourth threshold range by more than 1%. For example, if the minimum duty cycle of the fourth threshold range is 7%, more aggressive methods to increase the flow of data through the application layer may be used if the third transmission duty cycle is less than 6%. Less aggressive methods to increase the flow of data through the application may be used if the third transmission duty cycle is less than the fourth threshold range by less than 1%. For example, the minimum duty cycle of the fourth threshold range is 7%, more aggressive methods to increase the flow of data through the application layer may be used if the third transmission duty cycle is between 6% and 7%. Less aggressive methods to increase the flow of data through the application layer may include enabling the transmission of data within one or more lower-priority queues, increasing the frame rate at which video is transmitted, or similar methods. More aggressive methods to increase the flow of data through the application layer may include enabling the transmission through lower priority queues than less aggressive methods, enabling the transmission of queues that include large amounts of data such as videos, and other similar methods.

If the third transmission duty cycle D3 is not less than the fourth threshold at 1014, the method 1000 may proceed to 1012. At 1012, the method may include not changing the flow of data through the application layer. Similar to 1008, not changing the flow of data through the application layer may include continuing to transmit data from queues that were transmitting data when the method 1000 was initiated. Thus, the flow of data through the application layer may be increased if the third duty cycle D3 is below the fourth threshold range, the flow of data through the application layer may remain the same if the third duty cycle D3 is within the fourth threshold range, and the flow of data through the application layer may be decreased if the third duty cycle D3 is above the fourth threshold range.

An example of the path that data may take during transmission from a transmitting device (e.g., the patient monitor as disclosed herein) may begin the application layer. Transmission may be initiated at the application software within the application layer. For example, transmission may be initiated by an application executing on the transmitting device. In the example where the patient monitor 110 is the transmitting device, the data may be patient monitoring data (e.g., SpO2 values, respiration rate values, waveforms of SpO2 values, etc.). Transmission of the patient monitoring data may be initiated by an application on the patient monitor that receives the patient monitoring data from one or more sensors and processes the patient monitoring data for eventual transmission to a receiving device over a network. The application software may initiate the transmission of the data to the data control module. The data control module may control what data is transmitted from the application layer to the intermediate layers and the rate at which data is transmitted from the application layers to the intermediate layers. The data control module may sort the transmitted data into one or more priority-ranked queues, and transmit data from one or more of the one or more priority-ranked queues. The queues selected to transmit data and the rate at which data is transmitted from the application layer may be based on a comparison between the third duty cycle of the transmitter measured over the third time period relative to the fourth threshold range. The queues may be selected such that the data rate out of the application layer results in the third transmitter duty cycle remaining, at least on average, under the maximum value of the fourth threshold range. For example, if the third duty cycle is greater than the maximum value of the fourth threshold range, the waveforms generated by the application may not be released for eventual transmission, while the patient monitoring values (e.g., respiration rate values) may be released for eventual transmission, as the waveforms may be in a lower-priority queue than the patient monitoring values. Controlling the application layer in this manner may result in the third duty cycle remaining under the first threshold.

Data that flows out of the application layer (e.g., the patient monitoring values as explained above) may be processed by the intermediate layers before the data is passed to the network layer. The network layer may include the data rate limiter that may store the data (e.g., the patient monitoring values) in short term memory and control the rate of data out of the network layer by controlling the rate at which data is released from short term memory. The data rate limiter may be controlled based on the second duty cycle. For example, the maximum data rate at which the data is released from the short term memory may be set based on the second duty cycle; the maximum data rate may be decreased if the second duty cycle is above the second threshold range (e.g., above a maximum value of the second threshold range) and the maximum data rate may be decreased if the second duty cycle is below second threshold range (e.g., below a minimum value of the second threshold rang). In some examples, the data rate through the network layer is controlled by the data rate limiter to remain at or below a second data rate threshold (which may be the maximum value of the second threshold range).

Data from the network layer may be passed to the data link layer and the physical layer. The data at the physical layer may be controlled by the physical transmission controller (which may be a switch, as explained previously), which may block or allow the transmission of the data via the transmitter based on commands from the transmission controller. If the transmission of data is blocked, the data may be stored in short term storage within the memory of the transmission controller until transmission is allowed. The physical transmission controller may allow the transmission of data if the first duty cycle (e.g., measured over the first time period as explained above) is less than the first threshold and if the third duty cycle (e.g., measured over the third time period as explained above) is less than the third threshold. If transmission is allowed, the data (e.g., the patient monitoring values) may be transmitted via the transmitter to the network. Other mechanisms are possible for blocking the transmission of data via the transmitter, such as disabling an RF block in the transmitter (e.g., power amplifier, digital-to-analog converter, or modulator) or preventing all link layer transmission and retransmission.

The above disclosure provides support for an adaptive method of managing the radiated wireless energy by managing the transmission duty cycle of a radio transmitter. The mechanism combines measuring the transmission duty cycle continuously over various time periods with multi-layer control signaling for the transmitted data. Having control signaling on different layers of the communication protocol stack allows definition of both hard duty cycle limits-e.g. to force regulatory compliance-while optimizing transmitted data for better wireless coexistence and link performance.

The technical effect of implementing controls at multiple layers of the OSI model within a transmitter device responsive to one or more transmission duty cycles measured over one or more time periods is that the transmission duty cycle of the transmitter device can be controlled on different time scales and controlled using a plurality of methods, thereby reducing the severity and amount of interruptions to the transmission of data while the transmitter device is operating within regulatory limits.

In another representation, a method for a transmitter device includes adjusting a flow of data out of an application executing on the transmitter device based on a third duty cycle of a transmitter of the transmitter device, to maintain the third duty cycle within a fourth threshold range; releasing the data to the transmitter at a data rate that is equal to or less than a maximum data rate, the maximum data rate set based on a second duty cycle of the transmitter; and determining that a first duty cycle of the transmitter is less than a first threshold and that the third duty cycle of the transmitter is less than a third threshold, and in response, transmitting, with the transmitter, the data to a receiving device via a network.

The disclosure also provides support for a method for a transmitter, comprising: measuring a duty cycle of the transmitter over each of one or more time periods, and adjusting output of the transmitter based on each measured duty cycle relative to one or more respective thresholds, wherein the adjusting includes adjusting output of the transmitter at a physical or data link layer, adjusting output of the transmitter by adjusting a data rate of data released to the transmitter, and/or adjusting output of the transmitter by adjusting a data flow to the transmitter. In a first example of the method, adjusting the output of the transmitter based on each measured duty cycle relative to the one or more respective thresholds comprises adjusting the output of the transmitter at the physical or data link layer based on a first duty cycle relative to a first threshold, the first duty cycle measured over a first time period. In a second example of the method, optionally including the first example, adjusting the output of the transmitter based on each measured duty cycle relative to the one or more respective thresholds comprises adjusting the data rate of data released to the transmitter based on a second duty cycle relative to one or more second thresholds, the second duty cycle measured over a second time period. In a third example of the method, optionally including one or both of the first and second examples, adjusting the output of the transmitter based on each measured duty cycle relative to the one or more respective thresholds comprises adjusting the data flow to the transmitter based on a third duty cycle relative to a third threshold range, the third duty cycle measured over a third time period. In a fourth example of the method, optionally including one or more or each of the first through third examples, adjusting the output of the transmitter includes adjusting the data rate of data released to the transmitter, including decreasing a maximum data rate of the transmitter responsive to a respective duty cycle being greater than one of the one or more respective thresholds, and increasing the maximum data rate responsive to the respective duty cycle being less than another of the one or more respective thresholds. In a fifth example of the method, optionally including one or more or each of the first through fourth examples, adjusting the output of the transmitter includes adjusting the output of the transmitter at the physical or data link layer by blocking or allowing a radio frequency (RF) signal to an antenna. In a sixth example of the method, optionally including one or more or each of the first through fifth examples, adjusting the output of the transmitter includes adjusting the data rate of data released to the transmitter by controlling a data rate limiter to adjust a rate at which data stored in short term memory is released from the short term memory to the transmitter. In a seventh example of the method, optionally including one or more or each of the first through sixth examples, adjusting the output of the transmitter includes adjusting the data flow to the transmitter by, for a selected queue of data, adjusting a frame rate of data in the selected queue sent to the transmitter, adjusting a sampling rate of data in the selected queue sent to the transmitter, and/or stopping or allowing transmission of data from the selected queue to the transmitter. In an eighth example of the method, optionally including one or more or each of the first through seventh examples, the selected queue of data is one of a plurality of queues of data, wherein each queue of data is assigned a priority level and the selected queue of data has a lower priority level than one or more other queues of data of the plurality of queues of data. In a ninth example of the method, optionally including one or more or each of the first through eighth examples, adjusting the output of the transmitter based on each measured duty cycle relative to the one or more respective thresholds comprises adjusting the output of the transmitter at the physical or data link layer based on a third duty cycle relative to a fourth threshold, the third duty cycle measured over a third time period. In a tenth example of the method, optionally including one or more or each of the first through ninth examples, the first time period is shorter than the second time period and the second time period is shorter than the third time period.

The disclosure also provides support for a transmitter device, comprising: a transmitter, and a transmission controller including one or more processors and instructions stored in memory and executable by the one or more processors to: calculate a respective duty cycle of the transmitter over each of one or more time periods, adjust a flow of data through one or more layers of a transmitter model of the transmitter device based on each respective duty cycle, and transmit the data via the transmitter. In a first example of the transmitter device, adjusting the flow of data through the one or more layers of the transmitter model of the transmitter device based on each respective duty cycle comprises adjusting a flow of data through a physical layer of the transmitter device based on one or more respective duty cycles. In a second example of the transmitter device, optionally including the first example, adjusting the flow of data through the one or more layers of the transmitter model of the transmitter device based on each respective duty cycle comprises adjusting a flow of data through a network layer of the transmitter device based on one or more respective duty cycles. In a third example of the transmitter device, optionally including the first example and/or second example, adjusting the flow of data through the network layer of the transmitter comprises controlling a data rate limiter configured to control a rate at which data in short term storage is released from the short term storage. In a fourth example of the transmitter device, optionally including one or more or each of the first through third examples, adjusting the flow of data through the one or more layers of the transmitter model of the transmitter device based on each respective duty cycle comprises adjusting a flow of data through an application layer of the transmitter device based on one or more respective duty cycles. In a fifth example of the system, optionally including one or more or each of the first through fourth examples, adjusting the flow of data through the application layer of the transmitter comprises controlling a data control module configured to control transmission of one or more priority-ranked queues of data. In a sixth example of the transmitter device, optionally including one or more or each of the first through fifth examples, the transmitter device is a patient monitor, wherein adjusting the flow of data through the one or more layers of the transmitter model comprises adjusting a flow of patient monitoring data through the one or more layers of the transmitter model, the patient monitoring data received from one or more sensors coupled to a patient, and wherein transmitting the data comprises transmitting the patient monitoring data, via the transmitter, over a hospital network to one or more receiving devices.

The disclosure also provides support for a system, comprising: a network, and a transmitter device in communication with a receiving device via the network, the transmitter device comprising: a transmitter, a radio frequency (RF) power detector, and a transmission controller configured to control the transmitter to transmit data to the receiving device via the network based on a respective duty cycle of the transmitter calculated over each of a plurality of time periods, each respective duty cycle calculated based on output from the RF power detector. In a first example of the system, the transmission controller is configured to control the transmitter by commanding a physical or logical switch to block transmission of the data via the transmitter in response to a first duty cycle being greater than a first threshold and/or in response to a third duty cycle being greater than a third threshold, wherein the first duty cycle is calculated over a first time period and the third duty cycle is calculated over a third time period, the third time period greater than the first time period, and wherein the first threshold is higher than the third threshold. In a second example of the system, optionally including the first example, the transmission controller is configured to control the transmitter by commanding a data rate limiter to adjust a maximum data rate of data released to the transmitter in response to a second duty cycle being outside a second threshold range, wherein the second duty cycle is calculated over a second time period, the second time period greater than the first time period and less than the third time period, and wherein the second threshold range has a maximum value that is less than the first threshold and has a minimum value that is greater than the third threshold. In a third example of the system, optionally including one or both of the first and second examples, the transmission controller is configured to control the transmitter by commanding an application executing on the transmitter device to reduce a flow of data out of the application in response to the third duty cycle being greater than a fourth threshold, wherein the fourth threshold is less than the third threshold, and wherein the data that flows out of the application is transmitted by the transmitter at a data rate equal to or less than the maximum data rate.

When introducing elements of various embodiments of the present disclosure, the articles "a," "an," and "the" are intended to mean that there are one or more of the elements. The terms "first," "second," and the like, do not denote any order, quantity, or importance, but rather are used to distinguish one element from another. The terms "comprising," "including," and "having" are intended to be inclusive and mean that there may be additional elements other than the listed elements. As the terms "connected to," "coupled to," etc. are used herein, one object (e.g., a material, element, structure, member, etc.) can be connected to or coupled to another object regardless of whether the one object is directly connected or coupled to the other object or whether there are one or more intervening objects between the one object and the other object. In addition, it should be understood that references to "one embodiment" or "an embodiment" of the present disclosure are not intended to be interpreted as excluding the existence of additional embodiments that also incorporate the recited features.

In addition to any previously indicated modification, numerous other variations and alternative arrangements may be devised by those skilled in the art without departing from the spirit and scope of this description, and appended claims are intended to cover such modifications and arrangements. Thus, while the information has been described above with particularity and detail in connection with what is presently deemed to be the most practical and preferred aspects, it will be apparent to those of ordinary skill in the art that numerous modifications, including, but not limited to, form, function, manner of operation and use may be made without departing from the principles and concepts set forth herein. Also, as used herein, the examples and embodiments, in all respects, are meant to be illustrative only and should not be construed to be limiting in any manner.

## Claims

1. A method for a transmitter, comprising:
measuring a duty cycle of the transmitter over each of one or more time periods; and
adjusting output of the transmitter based on each measured duty cycle relative to one or more respective thresholds, wherein the adjusting includes adjusting output of the transmitter at a physical layer or data link layer, adjusting output of the transmitter by adjusting a data rate of data released to the transmitter, and/or adjusting output of the transmitter by adjusting a data flow to the transmitter.

2. The method of claim 1, wherein adjusting the output of the transmitter based on each measured duty cycle relative to the one or more respective thresholds comprises adjusting the output of the transmitter at the physical layer or data link layer based on a first duty cycle relative to a first threshold, the first duty cycle measured over a first time period.

3. The method of claim 1, wherein adjusting the output of the transmitter based on each measured duty cycle relative to the one or more respective thresholds comprises adjusting the data rate of data released to the transmitter based on a second duty cycle relative to one or more second thresholds, the second duty cycle measured over a second time period.

4. The method of claim 1, wherein adjusting the output of the transmitter based on each measured duty cycle relative to the one or more respective thresholds comprises adjusting the data flow to the transmitter based on a third duty cycle relative to a third threshold range, the third duty cycle measured over a third time period.

5. The method of claim 1, wherein adjusting the output of the transmitter includes adjusting the data rate of data released to the transmitter, including decreasing a maximum data rate of the transmitter responsive to a respective duty cycle being greater than one of the one or more respective thresholds, and increasing the maximum data rate responsive to the respective duty cycle being less than another of the one or more respective thresholds.

6. The method of claim 1, wherein adjusting the output of the transmitter includes adjusting the output of the transmitter at the physical layer or data link layer by blocking or allowing a radio frequency (RF) signal to an antenna of the transmitter.

7. The method of claim 1, wherein adjusting the output of the transmitter includes adjusting the data rate of data released to the transmitter by controlling a data rate limiter to adjust a rate at which data stored in short term memory is released from the short term memory to the transmitter.

8. The method of claim 1, wherein adjusting the output of the transmitter includes adjusting the data flow to the transmitter by, for a selected queue of data, adjusting a frame rate of data in the selected queue sent to the transmitter, adjusting a sampling rate of data in the selected queue sent to the transmitter, and/or stopping or allowing transmission of data from the selected queue to the transmitter, wherein the selected queue of data is one of a plurality of queues of data, and wherein each queue of data is assigned a priority level and the selected queue of data has a lower priority level than one or more other queues of data of the plurality of queues of data.

9. A transmitter device, comprising:
a transmitter; and
a transmission controller including one or more processors and instructions stored in memory and executable by the one or more processors to:
calculate a respective duty cycle of the transmitter over each of one or more time periods;
adjust a flow of data through one or more layers of a transmitter model of the transmitter device based on each respective duty cycle; and
transmit the data via the transmitter.

10. The transmitter device of claim 10, wherein adjusting the flow of data through the one or more layers of the transmitter model of the transmitter device based on each respective duty cycle comprises adjusting a flow of data through a physical layer of the transmitter device based on one or more respective duty cycles.

11. The transmitter device of claim 10, wherein adjusting the flow of data through the one or more layers of the transmitter model of the transmitter device based on each respective duty cycle comprises adjusting a flow of data through a network layer of the transmitter device based on one or more respective duty cycles.

12. The transmitter device of claim 12, wherein adjusting the flow of data through the network layer of the transmitter comprises controlling a data rate limiter configured to control a rate at which data in short term storage is released from the short term storage.

13. The transmitter device of claim 10, wherein adjusting the flow of data through the one or more layers of the transmitter model of the transmitter device based on each respective duty cycle comprises adjusting a flow of data through an application layer of the transmitter device based on one or more respective duty cycles.

14. The transmitter device of claim 14, wherein adjusting the flow of data through the application layer of the transmitter comprises controlling a data control module configured to control transmission of one or more priority-ranked queues of data.

15. The transmitter device of claim 10, wherein the transmitter device is a patient monitor, wherein adjusting the flow of data through the one or more layers of the transmitter model comprises adjusting a flow of patient monitoring data through the one or more layers of the transmitter model, the patient monitoring data received from one or more sensors coupled to a patient, and wherein transmitting the data comprises transmitting the patient monitoring data, via the transmitter, over a hospital network to one or more receiving devices.
